# FASCICULE DE BREVET EUROPEEN

(11) **EP 0 787 135 B1**
(45) Date de publication et mention de la délivrance du brevet: **24.05.2000**
(21) Numéro de dépôt: 95935993.6
(22) Date de dépôt: 20.10.1995
(51) Int. Cl.: C07H 17/04, A61K 31/70

(54) **DERIVES HYDROSOLUBLES D'EPIPODOPHYLLOTOXINE, LEUR PROCEDE DE PREPARATION, LEUR UTILISATION A TITRE DE MEDICAMENT, ET LEUR UTILISATION DESTINEE AUX TRAITEMENTS ANTICANCEREUX**
WASSERLÖSLICHE DERIVATE EPIPODOPHYLLOTOXINE, VERFAHREN ZU IHRER HERSTELLUNG, IHRE VERWENDUNG ALS MEDIKAMENT UND IHRE VERWENDUNG ZUR HERSTELLUNG EINES ANTIKREBSMITTELS
WATER-SOLUBLE EPIPODOPHYLLOTOXIN DERIVATIVES, PREPARATION METHOD THEREFOR, AND USE THEREOF AS A DRUG AND FOR TREATING CANCER

(30) Priorité: 21.10.1994 FR 9412597
(43) Date de publication de la demande: 06.08.1997
(73) Titulaire: PIERRE FABRE MEDICAMENT, 92100 Boulogne (FR)
(72) Inventeur: IMBERT, Thierry, F-81290 Viviers-les-Montagnes (FR); GUMINSKI, Yves, F-81090 Lagarrigue (FR); MONSE, Barbara, F-81100 Castres (FR); HILL, Bridget, F-81100 Castres (FR); ROBIN, Jean-Pierre, F-72000 Le Mans (FR)
(74) Mandataire: Ahner, Francis
(86) Numéro de dépôt international: FR9501388
(87) Numéro de publication internationale: WO9612727

(56) Documents cités:
- EP-A- 0 415 453
- EP-A- 0 445 021
- WO-A-94/14829
- GB-A- 2 207 674

## Description

Parmi la classe des épipodophylloïdes, certains composés comme l'étoposide ou le téniposide, dérivés de l'épipodophyllotoxine, composés hémi-synthétiques, issus de lignane naturel, sont utilisés dans la préparation de médicaments pour traiter de nombreuses formes de cancer. Ils sont considérés, actuellement comme des produits majeurs de l'arsenal thérapeutique.

Parmi les différents cancers traités par ce type de composés on peut citer le cancer du poumon à petites cellules, les tumeurs embryonnaires, neuroblastomes, cancer du rein, lymphomes, maladie de Hodgkin, leucémies aigües, et même cancer du sein. L'étoposide est avantageusement utilisé en association avec d'autres produits anticancéreux et en particulier les dérivés du Platine comme le Cis Platine.

L'inconvénient important de ce dérivé, et de même pour son dérivé apparenté le téniposide, est son manque d'hydrosolubilité. Il n'existe pas de formes commercialisées hydrosolubles pour des administrations intraveineuses. Au contraire la mise en solution est faite actuellement dans des solvants partiellement non aqueux, nécessite une administration en perfusion lente et provoque certains effets indésirables voire même toxiques. Il existe ainsi un besoin de formes hydrosolubles pour des produits dérivés de cette classe de composés, afin d'améliorer l'administration chez la malade, ainsi que sa biodisponibilité. La présente invention concerne donc des dérivés de l'étoposide hydrosolubles grâce à la présence de groupements fonctionnels phosphates ou carboxylates dont les sels d'addition organiques ou minéraux forment des entités solubles dans l'eau. Cette formulation aqueuse présente l'avantage d'être moins toxique et plus facilement administrable que les formes commercialisées actuellement.

La préparation de dérivés de l'étoposide a suscité de nombreux travaux et de nombreux brevets, et en particulier des dérivés diesters 2", 3" et triesters 2", 3", 4' d'étoposide ont été revendiqués dans le brevet FR 2 699 535-A1. Certains de ces dérivés ont montré une activité égale ou supérieure à l'étoposide et une moindre toxicité. Une amélioration supplémentaire est apportée maintenant grâce à une solubilité aqueuse qui leur confère une facilité d'administration et laisse présager une biodisponibilité accrue par un meilleur passage dans les différentes membranes biologiques.

La littérature mentionne des brevets relatifs à des composés apparentés à l'étoposide cherchant à améliorer l'hydrosolubilité, notamment (US 4 904 768, EP 0 369 369-A2, EP 0 196 618-A1, EP 0 320 988, EP 0 415 453-A2).

L'avantage de moduler la solubilité aqueuse des composés par des groupements phosphates a été utilisé dans quelques cas favorablement soit dans le domaine des anticancéreux WO 8707609, soit dans le domaine des analgésiques (BE 893,563) malgré tout rien ne laisse prévoir que le composé ainsi obtenu puisse conserver totalement une activité biologique intéressante en lui-même ainsi que celle du dérivé dont il est issu.

Il a été trouvé que les dérivés phosphates et carboxylates possèdent une hydrosolubilité permettant l'administration par voie injectable, et de plus manifestent une activité anticancéreuse améliorée par rapport à l'étoposide.

La présente invention concerne donc un composé de formule générale I 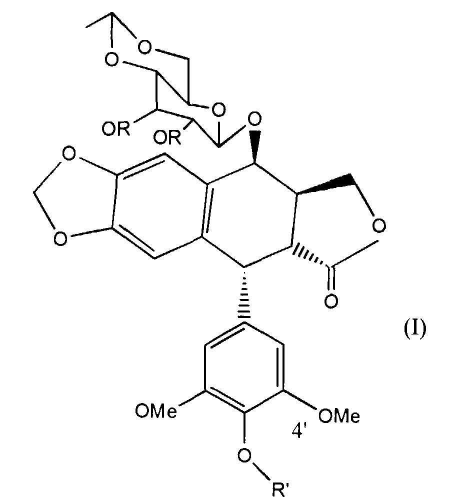 dans laquelle R' représente un groupement phosphate monoester ; un groupement carbamate du type -CO-N(R₁R₂) où N(R₁R₂) représente un groupement aminodiacétique ou une amine polycyclique comme la 3-aminoquinuclidine; un groupement phosphonoacétique CO-CH₂-PO₃H₂ ; CO-CH₂-SO₂CH₂COOH; CO-CH₂-OCH₂COOH; ou un radical R de formule A-Z-CH2-CO dans laquelle
- Z représente un atome d'oxygène, de soufre, un groupement SO₂, un alkylène linéaire ou ramifié en C₁₋₄, et
- A représente un noyau phényl substitué ou non à la condition que :
   - dans le cas où R' = R, c'est-à-dire les dérivés triacylés, A représente un noyau aromatique possédant une fonction salifiable, sauf 4-hydroxy-phényle,
   - dans le cas où R' ≠ R, A représente un reste benzyl, naphtyl, hétéroaryl, phényl substitué ou non, dans ce cas le phényl pouvant être substitué une, deux, trois, quatre ou cinq fois quelle que soit sa position sur le noyau aromatique par des groupes identiques ou différents choisis parmi les groupes halogènes, F, Cl, Br, alcoxy linéaire ou cycliques en C₁₋₆, alkyl C₁-C₆, méthylène dioxy, OCF₃, CF₃, NO₂, CN, OCH₂ Aryl, OH, OPO₃H₂, CH₂-PO₃H₂, PO₃H₂, OCH₂CO₂H, COOH, CH₂COOH, COCH₃, CHO,
ainsi que ses sels avec des acides ou des bases minérales ou organiques, thérapeutiquement acceptables et hydrosolubles.

De manière avantageuse les composés de formule générale I seront choisis avec R' représentant un groupe phosphate monoester (PO₃H₂), carbamate CONR₁R₂ et NR₁R₂ représentant un groupement aminodiacétique ou une amino-3 quinuclidine, R' représentant également un groupe phosphonoacétique CO-CH₂-PO₃H₂.
acétyl, phénylsulfonylacétyl, pentafluorophénoxyacétyl, 2 et 4 formylphénoxyacétyl, 4-cyanophénoxyacétyl.

La présente invention concerne également les procédés de préparation d'un composé de formule I, représentés sur le schéma 1 (voie A), pour lequel on fait réagir un intermédiaire glycosylé de formule générale II. 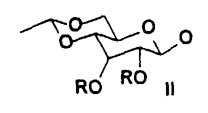 avec un intermédiaire de formule générale III pour fournir un intermédiaire IV 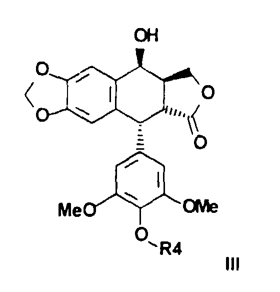 R ayant les significations précédentes. R₄ est un groupe protecteur par exemple le benzyloxycarbonyl ou un reste carbamate. Ce procédé de préparation est décrit dans le brevet antérieur FR 2 699 535 à l'exemple 17. Pour fournir un composé de formule IV dans lequel R₄ est un groupement protecteur et R défini précédemment. Ce dérivé IV est déprotégé sur sa position 4' (R₄), soit par hydrogénolyse, soit par hydrolyse faiblement basique pour fournir le dérivé I (R'=H). Il est également possible de préparer les composés de formule I où R' =R par cette méthode, en utilisant l'intermédiaire de formule III dans lequel R₄ représente un groupe acyl R, cette méthode est décrite également dans le brevet antérieur FR 2 699 535 à l'exemple 1. Selon les compatibilités des substituants R du glucosyl, il est possible également de synthétiser les composés de formule I à partir de l'étoposide lui-même (voie B).

Dans une première étape l'étoposide peut être protégé en position 4' (R₄) par un groupement R₄=benzyloxycarbonyl, ou par un groupement quinuclidine carbamate (R₄=CONH3-quinuclidinyl) obtenu par réaction successive du phosgène suivi de l'amino-3-Quinuclidine sur l'étoposide, pour fournir l'intermédiaire V.

D'une façon générale les intermédiaires V sont acylés par des chlorures d'acide dérivés des groupes R définis précédemment, (formés par action du chlorure d'oxalyl) en présence de pyridine, dans le chlorure de méthylène à basse température, sous réserve que les autres fonctions du groupe R soient inertes dans ces conditions, sinon, les substituants phénoliques, carboxyliques ou phosphoniques sont protégés sous forme d'éthers ou d'esters benzyliques respectivement, ce qui permet le déblocage, au stade suivant de la synthèse, par hydrogénolyse (V donnant I R'=H). Les dérivés pour lesquels R' =R sont préparés à partir de l'étoposide par triacylation sur les positions 2", 3" et 4' (voie C).

Dans le cas des dérivés fonctionnels R sensibles aux conditions d'hydrogénolyse comme par exemple mais non exclusivement, la présence de Cl ou de NO₂, le groupement protecteur choisi sur la position 4' peut être un dérivé carbamate de type CONH-3-quinuclidinyl ou d'un carbonate ou d'un ester de faible poids moléculaire, comme les chloroacétates qui peuvent se cliver ultérieurement entre autres dans des conditions faiblement basiques comme une solution aqueuse de bicarbonate de sodium à basse température sans influencer la stéréochimie de la lactone trans.

L'étape ultime I (R'=H) donnant I (R'≠H) consiste en une phosphorylation pour former un phosphate monoester du ou des phénols avec POCl₃ en présence de pyridine suivi d'une hydrolyse lente en milieu aqueux acide. L'obtention des dérivés possédant en position 4' un reste carbamate diacétique de type R' = CON (CH₂CO₂H)₂ se préparent par action du phosgène sur le composé de formule I (R' = H) pour former l'intermédiaire chlorocarbonate non isolé (R'=COCl) puis en le faisant réagir avec le diester benzylique de l'acide aminodiacétique suivi d'une hydrogénolyse pour libérer ensuite les fonctions acides sous forme libre.

Les dérivés phosphonoacétiques en position 4' s'obtiennent en faisant réagir le phénol libre en cette position avec le chlorure d'acide de l'acide diéthylphosphonoacétique (Synthesis 1978, 131) ou l'acide dibenzyl phosphonoacétique (Tet. Let. 1974, N°9, 711), puis en hydrolysant les fonctions esters phosphoniques par le bromure de triméthyl silyle, en présence de pyridine dans l'acétonitrile, dans le cas des esters éthyliques, ou par hydrogénolyse, dans le cas des esters benzyliques. Les dérivés de formule I où R'=R, c'est-à-dire possédant la même substitution acyle sur les positions 2", 3" et 4' s'obtiennent, par la voie C, par triacylation de l'étoposide lui-même avec des groupements acyles AZCH₂CO ayant une fonction carboxylique sur le groupement A défini précédemment, protégée par exemple sous forme d'ester benzylique, que l'on déprotège par hydrogénolyse ultérieurement.

Les dérivés carboxyliques, ou les dérivés phosphates ou phosphonates ainsi obtenus sont salifiés dans l'eau en présence éventuellement d'un cosolvant organique, par addition de bases organiques ou minérales en proportion stoechiométrique par rapport aux acidités en présence, et par exemple avec le N-méthylglucamine, la triéthanolamine, la lysine.

Les sels amorphes ou cristallisés sont obtenus par simple lyophylisation.

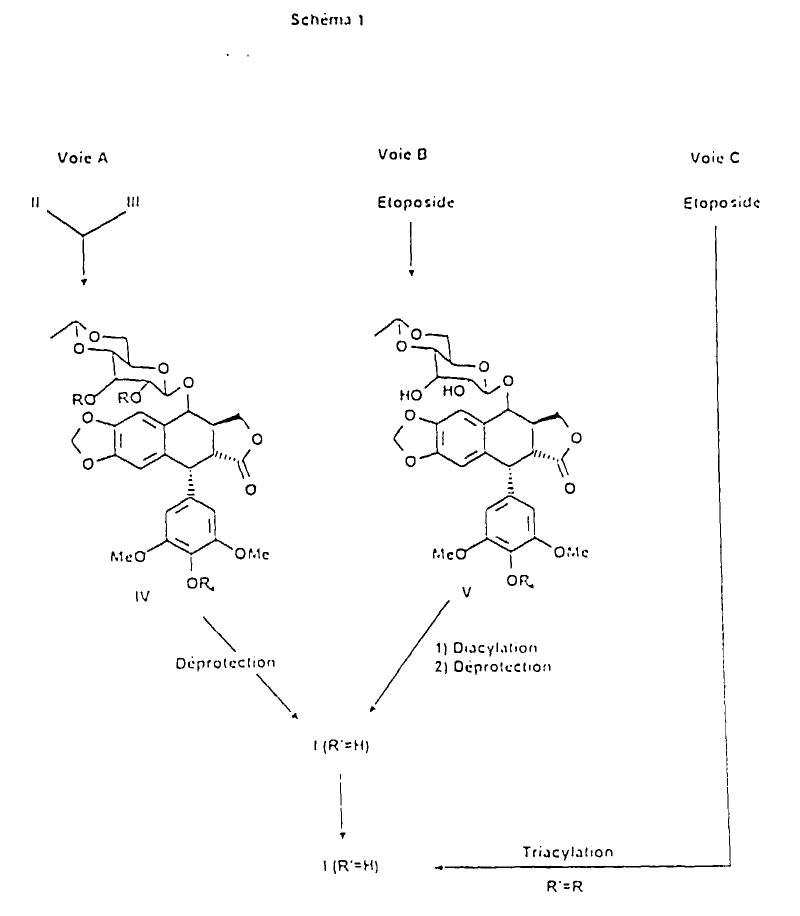

### Mesure de l'hydrosolubilité

La solubilité dans l'eau des sels des dérivés phosphates ou carboxylates par addition d'amines organiques physiologiquement acceptables comme par exemple la N-méthylglucamine, la triéthanolamine, la lysine, ou les sels avec des cations minéraux comme le sodium, obtenus sous forme lyophilisée ou par addition extemporanée d'une base sur le composé acide libre, a fourni les résultats suivants à titre d'exemple.

**Tableau II**

| **Composés** | **Sel** | **Solubilité % (poids/volume) exprimé en g pour 100ml d'eau** |
|---|---|---|
| Exemple 1 | N-méthylglucamine | 0,5 |
| Exemple 3 | N-méthylglucamine | 5 |
| Exemple 4 | N-méthylglucamine | 10 |
| Exemple 6 | N-méthylglucamine | 10 |
| Exemple 6 | triéthanolamine | 20 |
| Exemple 6 | lysine | 20 |
| Exemple 6 | Sodium | 1 |
| Exemple 7 | N-méthylglucamine | 10 |
| Exemple 11 | N-méthylglucamine | 10 |
| Exemple 19 | N-méthylglucamine | 10 |
| Exemple 24 | N-méthylglucamine | 10 |
| Exemple 25 | N-méthylglucamine | 0,5 |

Les dérivés ainsi préparés sont stables dans les conditions usuelles de pH neutre et acide et de température. Les dérivés phosphates sur la position 4' ont une stabilité chimique pour pouvoir se prêter aux différentes formulations pharmaceutiques.

### EXPERIMENTATION BIOLOGIQUE

Les molécules ont été testées in vitro en expérimentation biologique et ont montré leur intérêt en tant qu'agents anticancéreux dans les tests suivants. La mesure de l'inhibition de l'activité de la topoisomérase II est faite selon le protocole décrit dans la littérature : "Nuclear topoisomérase II levels correlate with the sensitivity of mammalian cells to intercalating Agents and Epipodophyllotoxins". I.D. HICKSON et coll., J. Biol. Chem. (1988), 263, 17724-1772.

Cette mesure a fourni les résultats suivants.

**Tableau I**

| **Composés** | **Test d'inhibition de l'activité de la topoisomérase II (ED**_{**50**} **M)** |
|---|---|
| Etoposide | 5.6 . 10⁻⁵ |
| Etopofos | > 10⁻⁴ |
| Exemple 1 | 5.6 . 10⁻⁶ |
| Exemple 3 | 3.2 . 10⁻⁷ |
| Exemple 4 | 1.8 . 10⁻⁶ |
| Exemple 6 | 3.2 . 10⁻⁷ |
| Exemple 7 | 5.6 . 10⁻⁵ |
| Exemple 11 | 5.6 . 10⁻⁶ |
| Exemple 19 | 5.6 . 10⁻⁶ |
| Exemple 24 | 5.6 . 10⁻⁶ |
| Exemple 25 | 7.6 . 10⁻⁷ |

La comparaison de l'étoposide avec son analogue soluble 4'-phosphate : étopofos (US-4 904 768) montre une perte d'activité in vitro. Ici les composés de l'invention se trouvent aussi, sinon plus, actifs que l'étoposide. Les groupements R et R' définis précédemment confèrent aux composés de l'invention une augmentation d'un facteur 10 à 100 de l'inhibition de l'activité enzymatique in vitro par rapport à l'étoposide.

On peut apprécier, au vu de ces résultats, l'intérêt de composés ayant une activité anticancéreuse égale ou supérieure à celle de l'étoposide, et une moindre toxicité, sur différentes formes de cancers comme en particulier le cancer du poumon à petites cellules, les tumeurs embryonnaires, les neuroblastomes, le cancer du rein, les tumeurs pédiatriques, les lymphomes hodgkimiens et non hodgkimiens, les leucémies aigües, les choriocarcinomes placentaires, les adénocarcinomes mammaires.

Ces dérivés peuvent être utilisés également dans les pathologies induites par le papilloma virus humain ainsi que l'arthrite rhumatoïde associée ou non à des pathologies cancéreuses.

De plus ces dérivés peuvent être utilisés pour augmenter l'efficacité thérapeutique des composés inhibiteurs de topoisomérase II et en particulier le traitement des tumeurs normalement réfractaires à la thérapeutique usuelle, c'est-à-dire les cancers colorectaux et les mélanomes. De plus on peut apprécier l'intérêt de ces produits présentant, d'une part une importante hydrosolubilité qui permet une administration par voie intraveineuse et orale aisée, et d'autre part une meilleure biodisponibilité que celle de l'étoposide. La présente invention concerne également les compositions pharmaceutiques comprenant au moins un composé de formule générale I selon l'invention et un excipient approprié.

Les compositions pharmaceutiques peuvent être présentées de façon adaptée pour l'administration par voie injectable ou par voie orale sous forme de capsule, de gélules, de comprimés à la posologie de 2 à 200 mg/m² par voie injectable et de 5 à 400 mg/m² par 24 h pour la voie orale.

A titre d'exemple et de façon non limitative les exemples suivants décrivent la préparation des composés de l'invention :

### Exemple 1 - formule I

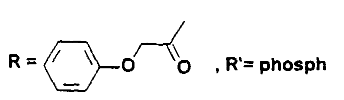

### 4'-déméthyl-4-O-(2,3-bis-phénoxyacétyl-4,6-éthylidène-β-D-glucosyl)-épipodophyllotoxine-4'-désoxy-4'-phosphate.

A une solution de 4'-déméthyl-4-O-(2,3-bis-phénoxyacétyl-4,6-ethylidène-β-D-glucosyl)-épipodophyllotoxine (1g, 1,16 mmole) dans 50 ml de THF à 10°C sont ajoutés 0,22 ml (2,33 mmoles) de POCl₃ puis 0,5 ml (3,5 mmoles) de triéthylamine. L'agitation est maintenue 30 mn à cette température. L'hydrolyse est effectuée ensuite par addition dans le milieu de 20 ml d'acide chlorhydrique N puis agité une nuit à température ambiante. Le milieu réactionnel est extrait avec l'acétate d'éthyle pour obtenir le dérivé phosphate cristallisant dans l'éther isopropylique avec un rendement quantitatif.

Les caractéristiques sont les suivantes :
F°C ∼ 175°C Anal. C₄₅H₄₅O₂₀P ; 1,5H₂O ; PM = 963,831

| | C | H |
|---|---|---|
| Calc.% | 56,07 | 5,02 |
| Tr. % | 56,18 | 4,73 |

Spectre de Masse (FAB) m/e 959 (M⁺ +Na)
1H 200MHz RMN CDCl₃ δ 1,30 (3H, d, J = 4,4Hz, H_{8"}) ; 2,9 (1H, m, H₃) ; 3,1 (1H, m, H₂) ; 5,0 (1H, dd, J = 8,8Hz, H₂") ; 5,3 (1H, dd, J ∼ 9,2Hz, H_{3"}) ; 5,5 (1H, s, OCH_{A}O) ; 5,7 (1H, s, OCH_{B}O) ; 6,25 (2H, s, H₂,H₆,) ; 6,44 (1H, s, H₈).
IR ν (KBr) 2941, 1774, 1599, 1487.

### Sel de N-méthyl glucamine

Le dérivé phosphate précédent est mis en suspension dans l'eau et on ajoute 2 équivalents de N-méthyl glucamine en solution 0,1M dans l'eau. La solution est agitée sous ultrasons et diluée à 200 ml. Après filtration la solution est glacée puis lyophilisée pendant 12 h. Le résidu est alors repris dans l'acétone et cristallisé, filtré séché pour fournir 350 mg d'un solide blanc F ≈ 135°C.
Anal. C₅₉H₇₉N₂O₃₀P, H₂O PM = 1345, 256

| | C | H | N |
|---|---|---|---|
| Calc. | 52,68 | 6,06 | 2,08 |
| Tr. | 52,69 | 5,86 | 1,68 |

IR ν (KBr) 3426, 1772, 1599, 1487.
RMN ¹H 200MHz CDCl₃ δ 1,22 (3H, d, J = 4,8Hz, H_{8"}) ; 2,3 (6H, s, N-CH₃), 2,6-3,0 (2H, m, H₂-H₃) ; 5,36 (1H, dd, J = 7,8Hz, H_{3"}) ; 5,74 (1H, s, OCH_{A}O) ; 5,97 (1H, s, OCH_{B}O) ; 6,15 (2H, s, H_{2'}-H_{6'}) ; 6,5 (1H, s, H₈) ; 7,10 (1H, s, H₅) ; 6,65 (2H, d, J = 8Hz, Ar Ortho) ; 6,8 à 6,96 (2H, d, J = 8Hz Ar ortho et 2H, t, J = 7Hz, Ar para) ; 7,24 (4H, m, Ar meta).

### Sel de Sodium

Le dérivé phosphate précédent est agité en solution dans l'acétone avec une résine échangeuse d'ion (Dowex 50 x 8 - 100) préparée par élution avec la soude N. Le milieu est allongé avec de l'eau, filtré et concentré. Le résidu aqueux est lyophillisé pour fournir le di-sel de sodium
F° ∼ 190°C Anal C₄₅H₄₃Na₂O₂₀P_{3.3}H₂O PM = 1040,208

| | C | H |
|---|---|---|
| Calc. | 51,96 | 4,81 |
| Tr. | 51,56 | 4,51 |

Par la même méthode que celle de l'exemple 1 mais en utilisant les composés intermédiaires de formule I (R' = H) correspondants les nouveaux dérivés suivants ont été préparés :

### Exemple 2 - formule I

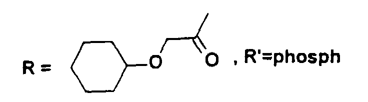

### 4'-déméthyl-4-O-(2,3-bis-cyclohexyloxyacétyl-4,6-éthylidène-β-D-glucosyl)-épipodophyllotoxine-4'-désoxy-4'-phosphate.

Rdt = 90 %
F° ∼ 160°CAnal. C₄₅H₄₅O₂₀P, H₂O PM = 966,920

| | C | H |
|---|---|---|
| Calc. | 55,89 | 6,15 |
| Tr. | 55,70 | 6,11 |

### Sel de N-méthylglucamine

### 4'-déméthyl-4-O-(2,3-bis-cyclohexyloxyacétyl-4,6-éthylidène-β-D-glucosyl)-épipodophyllotoxine-4'-désoxy-4'-phosphate, di-sel de N-méthylglucamine.

Rdt = 50 %
F° ∼ 112°C Anal. C₅₉H₉, N₂O₃₀P, 4H₂O PM = 1411,420

| | C | H | N |
|---|---|---|---|
| Calc. | 50,21 | 7,07 | 1,99 |
| Tr. | 50,16 | 6,60 | 2,30 |

### Exemple 3 - formule I

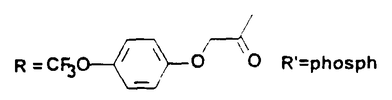

### 4'-déméthyl-4-O-(2,3-bis-(4-trifluorométhoxyphenoxyacétyl)-4,6-éthylidène-β-D-glucosyl)-épipodophyllotoxine-4'-désoxy-4'-phosphate, di-sel de N-méthyl glucamine.

Rdt = 68 %
F ∼ 132°C Anal. C₆₁H₇₇N₂O₃₂F₆P, 2,6H₂O PM = 1541,840

| | C | H | N |
|---|---|---|---|
| Calc. | 47,52 | 5,37 | 1,82 |
| Tr. | 47,15 | 5,51 | 2,11 |

### Exemple 4 - formule I

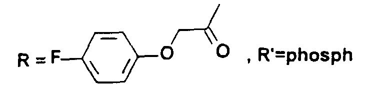

### 4'-déméthyl-4-O-(2,3-bis-(4-fluorophénoxyacétyl)-4,6-éthylidène-β-D-glucosyl)-épipodophyllotoxine-4'-désoxy-4'-phosphate di-sel de N-méthyl glucamine.

Rdt = 60 %
F ∼ 130°C Anal. C₅₉H77N₂O₃₀F₂P, 2,7 H₂O PM = 1363,240

| | C | H | N |
|---|---|---|---|
| Calc. | 50,17 | 5,88 | 1,98 |
| Tr. | 49,74 | 5,67 | 1,92 |

### Exemple 5 - formule I

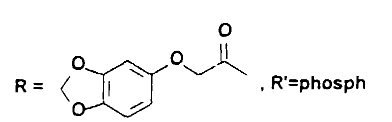

### 4'-déméthyl-4-O-(2,3-bis-(3,4-méthylènedioxyphenoxyacétyl)-4,6-éthylidène-β-D-glucoxyl)-épipodophyllotoxine-4'-désoxy-4'-phosphate di-sel de N-méthylglucamine.

Rdt = 50 %
F ∼ 120°C Anal. C₆₁H₇₉N₂O₃₄P, H₂O PM = 1433,274

| | C | H | N |
|---|---|---|---|
| Calc. | 51,08 | 5,70 | 1,95 |
| Tr. | 50,68 | 5,58 | 1,94 |

### Exemple 6 - formule I

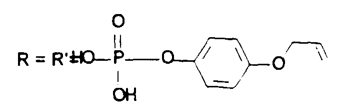

### 4'-(4-phosphonooxyphénoxyacétyl)-4'-déméthyl-4-O-(2,3-bis-(4-phosphonooxyphénoxyacétyl)-4,6-éthylidène-β-D-glucosyl)-épipodophyllotoxine.

Le dérivé phénolique de formule I correspondant à R = R' = 4-hydroxyphénoxyacétyl est décrit dans le brevet FR 2 699 535-A₁ à l'exemple 16 préparé par la voie A. 1g de ce dérivé (9,6 mmoles) est placé dans 50 ml de THF à -10°C sous atmosphère d'azote et on ajoute 1,2 ml (8,7 mmoles) de triéthylamine puis goutte à goutte 0,53 ml (5,8 mmoles) de POCl₃, l'agitation est maintenue 30 mn. Après filtration du chlorhydrate de triéthylamine formé, le THF est évaporé. Le résidu est repris dans HCl 1N et agité à température ambiante pendant 30 mn. Le précipité blanc est filtré, lavé à l'eau et séché sous vide à 60°C pendant une nuit. On obtient 800 mg de dérivé sous forme de phosphate libre. Rdt = 80 %.
F ∼ 160°C Anal. C₅₃H₅₃O₃₁P₃ PM = 1278,898
Spectre de masse (FAB) m/e : 1277 (M⁺-1)
IR (KBr) ν (cm-1) 3404, 1768, 1603, 1500, 1485, 1203, 1086.
RMN 1H 200Mz (DMSO) δ : 1,23 (3H, d, J = 4,26Hz H_{8"}) ; 3,03 (2H, m, H₂-H₃) ; 5,37 (2H, m, H_{2"}-H_{3"}) ; 5,77 (1H, s, O-CH_{A-O}) ; 5,97 (1H, s, O-CH_{B-O}) ; 6,3 (2H, s, H₂,-H_{6'}).

La préparation du sel de glucamine se fait de façon similaire à l'exemple 1 mais avec addition de 3 équivalents de N-méthylglucamine. Le composé s'obtient directement après lyophilisation, avec un rendement de 88 % donnant les analyses suivantes :
F ∼ 155°C Anal. C₇₄ H₁₀₄N₃O₄₆P₃ PM = 1864,54

| | C | H | N |
|---|---|---|---|
| Calc. | 47,67 | 5,62 | 2,25 |
| Tr. | 47,26 | 5,62 | 2,38 |

IR (KBr) ν (cm-1) : 3458, 1768, 1604, 1500, 1485, 1199, 1084.

### Exemple 7 - formule I

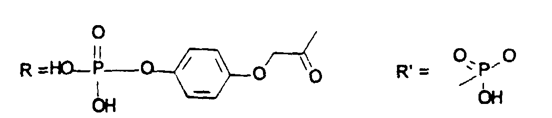

### 4'-déméthyl-4-O-(2,3-bis-(4-phosphonooxyphénoxyacétyl)-4,6-éthylidène-β-D-glucosyl)-épipodophyllotoxine-4'-désoxy-4'-phosphate.

Par la même suite de réactions que pour l'exemple 6 mais en utilisant le dérivé de formule I (R=4-hydroxyphénoxyacétyl et R'=H). Décrit dans le brevet FR 2 699 535-A1 à l'exemple 20, on obtient le composé avec un rendement de :
F ∼ 130°C Anal. C₆₆H₉₈ N₃O₄₃P₃, 6H₂O PM = 1822, 503

| | C | H | N |
|---|---|---|---|
| Calc. | 43,49 | 6,08 | 2,31 |
| Tr. | 43,30 | 5,88 | 2,77 |

IR (KBr) ν (cm-1) : 3429, 1763, 1508, 1199, 1084.

### Exemple 8 - formule I

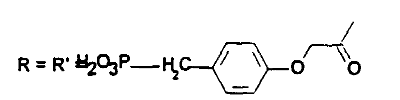

### 4'-(4-phosphonométhylphénoxyacétyl)-4'-déméthyl-4-O-(2,3-bis-(4-phosphonométhylphénoxyacétyl)-4,6-ethylidène-β-D-glucosyl)-épipodophyllotoxine.

### 1er stade : 4-benzyloxyphénylméthyldiéthylphosphonate

1g (4,3 10⁻³ moles) de chlorure de 4-benzyloxybenzyle sont portés 6 h au reflux avec 0,9 ml (5,15.10⁻³ moles) de triéthylphosphite. Le milieu réactionnel est filtré sur 150 g de SiO₂, éluée par un mélange d'heptane-acétate d'éthyle (20-80) pour fournir après évaporation 1,4 g du dérivé phosphonate (Rdt = 100 %).

### 2e stade : 4-hydroxyphénylméthyldiéthylphosphonate

Dans un autoclave, 1,1 g (3,3.10⁻³ moles) du dérivé benzyloxy du 1er stade sont hydrogénés sous une pression d'hydrogène de 7 bars en présence de 200 mg de charbon palladié à 10 % dans 15 ml d'un mélange d'acétate d'éthyle - éthanol (90-10) à une température de 80°C pendant 12 h sous agitation. Après filtration du catalyseur, le filtrat est évaporé sous pression réduite pour obtenir 800 mg (Rdt 100 %) du dérivé phénolique.

### 3e stade : Acide diéthyl phosphonométhylphénoxyacétique

A une solution THF (250 ml) de 2,7 g (11 mmoles) du dérivé phénolique précédent sont ajoutés 1,3 g (26 mmoles) de NaH (50 % dispersion) à température ambiante, puis 1,8 g (13 mmoles) d'acide bromoacétique sont introduits et le milieu réactionnel est porté 8 h au reflux. Le milieu réactionnel est versé sur 1 l d'eau glacé et extrait par l'éther éthylique. Les phases aqueuses sont acidifiées à pH 1.2 et extraites par l'acétate d'éthyle, séchées, évaporées pour fournir 3,1 g (Rdt 94 %) du dérivé acétique.
¹H 200MHz RMN (CDCl₃) δ 8.09 (massif 1H, échangeable) 7,16-7,26 (dd, 2H, J = 8Hz, 2Hz, H aromatiques), 6,85 (2H, d, J = 8Hz, H aromatiques), 4,6 (2H, s, OCH₂CO₂H), 4,0 (4H, m, ester phosphonate OCH₂), 3,12 (2H, d, J = 21,7Hz, CH₂P), 1,23 (6H, t, J = 7Hz, ester phosphonate OCH₂CH₃).

### 4e stade : condensation de l'acide obtenu au 3e stade sur l'étoposide

A 3 g (10,2 mmoles) d'acide précédemment obtenu au 3e stade en solution dans du chlorure de méthylène (15 ml) et 0,2 ml de DMF à 0°C sous azote, sont ajoutés goutte à goutte 1,4 g (11,2 mmoles) de chlorure d'oxalyle, après un important dégagement de CO₂ on laisse revenir le milieu réactionnel à température ordinaire. On refroidit de nouveau à 0°C pour introduire une solution contenant 1 g (1,7 mmole) d'étoposide, 2 g (25,5 mmoles) de pyridine dans du chlorure de méthylène (45 ml) goutte à goutte. En fin d'addition le milieu est agité encore pendant 4 h avec retour à température ordinaire. Après évaporation sous pression réduite le milieu réactionnel est repris par du toluène et évaporé, le résidu est agité avec de l'acétate d'éthyle et de l'acide chlorhydrique N. Après extraction, la phase organique est lavée par une solution glacée de bicarbonate de sodium puis par une solution saturée de NaCl, décantée, séchée, évaporée, elle fournit une mousse brune qui est chromatographiée sur SiO₂ (éluant CH₂Cl₂- MeOH-98-2) et donne après évaporation un résidu solide de 220 mg (Rdt 10 %), Masse (FAB) m/e 1441 (M⁺).
¹H RMN 200MHz fournit les pics caractéristiques de ces molécules (CDCl₃) δ 7,20 (6H, m, H arom-phosphonate), 6,65-6,93 (6H, d, J = 8,4Hz, H arom. phénoxy) 6,75 (1H, s, H₅), 6,47 (1H, s, H₈), 6,24 (2H, s, H_{2'} et H_{6'}), 5,87 (1H, s, OCH_{A}O), 5,61 (1H, s, OCH_{B}O), 5,35 (1H, t, H_{3"}), 5,05 (1H, t, H_{2"}), 3,0 (6H, d, J = 21Hz, CH₂ Phosphonate).

### 5e stade : Hydrolyse des esters phosphoniques

220 mg (0,16 mmoles) du dérivé triester phosphonique obtenu au 4e stade sont placés dans CH₃CN (50 ml) à 0°C sous azote, on additionne 0,26 ml de pyridine (3,2 mmoles) puis goutte à goutte 0,49 g (3,2 mmoles) de bromure de triméthylsilyle. L'agitation est maintenue 24 h avec retour à température ambiante. On évapore à sec, reprend le milieu par HCl N et le produit précipite, on filtre le précipité blanc, rince à l'eau jusqu'à neutralité. Le précipité est solubilisé dans du méthanol, filtré, évaporé. Le résidu est repris dans l'eau, cristallisé, pour fournir 120 mg du dérivé phosphonique (Rdt 57%).

F° = 190°C Anal C₅₆ H₅₉ O₂₈ P₃, 5H₂O PM = 1301, 41

| | C | H |
|---|---|---|
| Calc % | 51,68 | 5,34 |
| Tr % | 51,91 | 4,90 |

IR ν (KBr) 3431, 2922, 1774, 1608, 1512, 1485
¹H 200MHz RMN (CD₃OD) δ 7,15-7,23 (6H, m, H arom. méthyl phosphonique), 7,04 (1H, s, H₅), 6,9 (2H, d, H arom phénoxy), 6,8 (2H, d, H arom phénoxy), 6,67 (2H, d, H arom. phénoxy), 6,50 (1H, s, H₈), 6,35 (2H, s, H_{2'}, H_{6'}), 5,85 (1H, s, OCH_{A}O), 5,58 (1H, s, OCH_{B}O), 3,05 (2H, d, CH₂P), 2,9 (1H, dd, H₃), 1,3 (3H, d, H_{8"}).

Par la même réaction que pour l'exemple 8 4e stade ou l'exemple 25, on prépare (voie C) par triacylation sur l'étoposide les composés suivants à partir des acides A-Z-CH₂-CO₂H correspondants :

### Exemple 9 - formule I

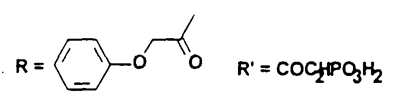

### 4'-(phosphonoacétyl)-4'-déméthyl-4-O-(2,3-bis-phénoxyacétyl-4,6-éthylidène-β-D-glucosyl)-épipodophyllotoxine.

A une solution de 400 mg (2 mmoles) d'acide diéthyl phosphonoacétique dans 5 ml de CH₂Cl₂ et 3 gouttes de DMF sous azote à 0°C, sont ajoutés 266 mg (2,1 mmoles) de chlorure d'oxalyle. L'agitation est maintenue 15 mn à 0°C, puis une solution de 500 mg (0,583 mmoles) de 4'-déméthyl-4-0-(2,3-bis-phénoxyacétyl-4,6-éthylidène-β-D-glucosyl)-épipodophyllotoxine dans 5 ml de CH₂Cl₂ et 184 mg (188 µl, 23 mmoles) de pyridine sont introduits au milieu réactionnel à 0°C. Le contact est maintenu 2,5 h, puis le milieu réactionnel est versé sur HCl N. La phase organique est décantée, lavée avec une solution de NaCl, séchée, évaporée. Le résidu est cristallisé dans l'éther éthylique pour fournir un précipité blanc (450 mg, Rdt 75 %).

320 mg (0,31 mmoles) de ce dérivé sont placés sous agitation dans 10 ml d'acétonitrile en présence de 470 mg (0,4 ml, 0,31 mmoles) de bromure de triméthyl silyle et 240 mg (0,25 ml, 0,31 mmoles) de pyridine, à température ambiante pendant 6 h.

Après évaporation le résidu est repris dans HCl N pour fournir un solide blanc qui est filtré, lavé à l'eau, séché. On obtient 180 mg (Rdt 57 %) du dérivé phosphonique
F° ∼ 140°C Anal C₄₇ H₄₇ O₂₁ P, 2H20 (PM = 1014, 996)

| | C | H |
|---|---|---|
| Calc % | 55,61 | 5,06 |
| Tr % | 55,87 | 4,80 |

IR ν (KBr) 3431, 1774, 1601, 1487
¹H 200MHz RMN (CDCl₃) δ 7,16-7,27 (4H, m, H m. Arom., 6,68-6,95 (7H, m, H o.p. Arom, H₅), 6,44 (1H, s, H₈), 6,24 (2H, s, H_{2'}-H_{6'}), 5,82 (1H, s, OCH_{A}O), 5,56 (1H, s, OCH_{B}O), 5,32 (1H, dd, H_{3"}), 5,02 (1H, t, H_{2"}), 3,2 (m, 3H, CH₂P, H₂), 1,32 (d, 3H, J - 4.4Hz, H_{8"}).

### Exemple 10 - formule I

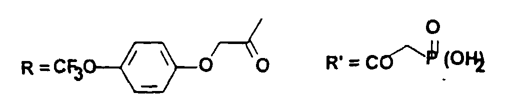

### 4'-(phosphonoacétyl)-4'-déméthyl-4-O-(2,3-bis-(4-trifluorométhoxyphénoxyacétyl)-4,6-éthylidène-β-D-glucosyl)-épipodophyllotoxine. Sel de N-méthyl glucamine.

Ce dérivé est obtenu par la même méthode que pour l'exemple 9 mais en partant du dérivé de formule I pour lequel R=4-trifluorométhoxyphénoxyacétyl et R' =H.

Préparation du sel de N-méthyl glucamine : on introduit 810 mg (0,7 mmoles) du dérivé phosphonique dans l'éthanol (15 ml) et 0,5 ml d'acétone puis 14,1 ml d'une solution 0,1N de N-méthyl glucamine (1,41 mmoles) dans l'éthanol sont introduits goutte à goutte sous agitation. L'agitation est maintenue 1 h. Le milieu réactionnel est évaporé et le résidu repris dans l'eau puis filtré (filtre de 0,45 µ). La solution aqueuse est lyophylisée, et le résidu est repris dans l'isopropanol, cristallisé, filtré, séché pour fournir 740 mg (Rdt 65 %) de sel de N-méthyl glucamine.
F° = 120°C Anal C63 H79 N2 F6 033 P, 3,5H2O PM = 1600.47

| | C | H | N |
|---|---|---|---|
| Calc % | 47,28 | 5,42 | 1,75 |
| Tr % | 46,95 | 5,47 | 2,07 |

IR ν (KBr) 3426, 1774, 1601, 1500, 1487

### Exemple 11 - formule I

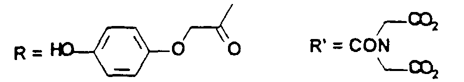

### 4'-(dicarboxyméthylaminocarbonyl)-4'-déméthyl-4-O-(2,3-bis-(p-hydroxy-phénoxyacétyl)-4,6-éthylidène-β-D-glucosyl)-épipodophyllotoxine.

### 1er stade : préparation du diesterbenzylique de l'acide aminodiacétique

A une solution de 10 g (49,6 mmoles) de chlorhydrate de l'ester benzylique de la glycine dans 200 ml de CH₃CN sont ajoutés 13,7 g (99 mmoles) de K₂CO₃, et 8 ml (49,6 mmoles) de bromoacétate de benzyle goutte à goutte. Le milieu est agité à température ambiante pendant 2 jours. 200 ml d'eau sont ajoutés et le milieu est acidifié à pH 2-3 par HCl concentré, puis extrait par l'acétate d'éthyle, pour obtenir 12 g (Rdt 80 %) du diester utilisé dans l'étape suivante.

### 2e stade : préparation du carbamate

Une solution de phosgène (0,79 ml, 1,5 mmole) dans le toluène à 1,93 M est introduite dans 50 ml d'acétonitrile puis refroidie à - 10°C sous atmosphère d'azote. On introduit goutte à goutte 820 mg (0,76 mmoles) de 4'-démetéhyl-4-0-(2,3-bis-(p-benzyloxyphénoxyacétyl)-4,6-éthylidène-β-D-glucosyl)-épipodophyllotoxine dans 13 ml d'acétonitrile et 0,24 g de diisopropyléthylamine. Le milieu réactionnel est agité 2 h à -10°C, puis on y introduit 0,24 g du diester benzylique de l'acide aminodiacétique, obtenu au 1er stade, dans 6 ml d'acétonitrile à - 5°C. L'agitation est maintenue 6 h. Le milieu réactionnel est ensuite évaporé puis filtré sur SiO₂ et élué avec un gradient de solvant : éther de pétrole - acétate d'éthyle 70.30 puis 60.40 et enfin 50.50 pour obtenir 700 mg (Rdt 65 %) du dérivé diester benzylique.

### 3e stade : Hydrogénolyse des fonctions benzyliques

700 mg du dérivé obtenu au 2e stade sont placés en solution dans un mélange de 13 ml d'acétate d'éthyle et 3 ml d'éthanol, dans un autoclave sous atmosphère d'hydrogène en présence de 70 mg de charbon palladié à 10 %. L'agitation est maintenue pendant 24 h puis le milieu réactionnel est filtré, évaporé. Le résidu est cristallisé dans l'éther isopropylique pour obtenir 480 mg (Rdt 92 %) du dérivé diacide.
F° - 150°C Anal C₅₀ H₄₉ NO₂₄ PM = 1047,94

| | C | H | N |
|---|---|---|---|
| Calc % | 57,31 | 4,71 | 1,34 |
| Tr % | 57,07 | 4,96 | 1,13 |

IR ν (KBr) 3433, 1768, 1603, 1512, 1485, 1460, 1236, 1199
¹H 200MHz RMN (DMSO) δ 9,03 et 8,98 (2H, 2s, CO₂H, échangeables), 6,45-6,67 (10H, m, H₅, H₈, ArH), 6,21 (2H, s, H_{2'}, H_{6'}), 6,0 (1H, s, OCHAO), 5,79 (1H, s, OCH_{B}O), 5,32 (2H, m, H_{2"} et H_{3"}), 3,39 (s, N-CH₂-CO₂H), 1,20 (3H, d, H_{8"}).

Par la même méthode que l'exemple 11 - 2e stade, mais en utilisant les composés intermédiaires de formule I (R' = H) correspondants, les nouveaux dérivés suivants ont été préparés.

### Exemple 12 - formule I

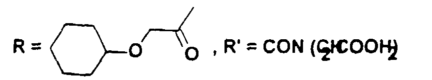

### 4'-(dicarboxyméthylaminocarbonyl)-4'-déméthyl-4-O-(2,3-bis cyclohexyloxy acétyl-4,6-ethylidène-β-D-glucosyl)-épipodophyllotoxine.

Rdt = 98 %
F° = 170°C Anal C₅₀ H₆₁ NO₂₂ PM = 1028,037

### Exemple 13 - formule I

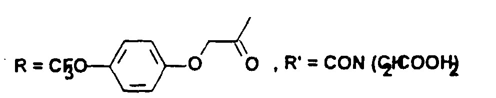

### 4'-(dicarboxyméthylaminocarbonyl)-4'-déméthyl-4-O-(2,3-bis(p-trifluorométhoxy phénoxy acétyl)-4,6-éthylidène-β-D-glucosyl)-épipodophyllotoxine.

Rdt = 87 %
F ∼ 150°C Anal C₅₂ H₄₇ NO₂₄ F₆ PM = 1183,94

| | C | H | N |
|---|---|---|---|
| Calc % | 52,75 | 4,00 | 1,20 |
| Tr % | 52,64 | 4,10 | 1,30 |

### Exemple 14 - formule I

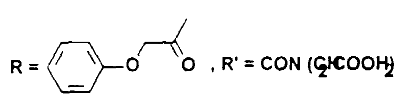

### 4'-(dicarboxyméthylaminocarbonyl)-4'-déméthyl-4-O-(2,3-bis phénoxyacétyl-4,6-éthylidène-β-D-glucosyl)-épipodophyllotoxine.

Rdt = 25 %
F ∼ 170°C Anal C₅₀ H₄₉ N O₂₂, H₂O PM = 1033,955

| | C | H | N |
|---|---|---|---|
| Calc % | 58,08 | 4,97 | 1,35 |
| Tr % | 58,43 | 5,05 | 1,28 |

### Exemple 15 - formule I

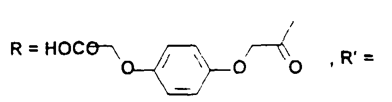

### 4'-déméthyl-4-O-(2,3-bis-(4-carboxyméthoxyphénoxyacétyl)-4,6-éthylidène-β-D-glucosyl)-épipodophyllotoxine, sel de N-méthylglucamine. 1er stade : 4-hydroxyphénoxyacétate de benzyle

A une suspension de 10 g (59 mmoles) d'acide p-hydroxyphénoxyacétique dans 100 ml de CH₂Cl₂ à 0°C sous atmosphère d'azote sont ajoutés 0,2 ml de DMF puis goutte à goutte 9 g de chlorure d'oxalyle (71 mmoles). L'agitation est maintenue 12 h à température ordinaire, puis 7,7 g (71 mmoles) d'alcool benzylique sont introduits et l'agitation est gardée 8 h à température ambiante. On jette le milieu réactionnel sur une solution ammoniacale glacée et on extrait par du chlorure de méthylène. La phase organique est lavée avec HCl N, décantée, séchée, évaporée. Le résidu est filtré sur 150 g de SiO₂ et éluée par un mélange heptane - acétate d'éthyle (75-25) pour fournir 3,8 g (Rdt 25 %) d'un solide blanc.
¹H 200MHz RMN (CDCl₃) δ 7,36 (5H, s, Ar), 6,75 (4H, d, ArOH), 5,24 (2H, s, CH₂ Ar), 4,61 (2H, s, OCH₂CO).

### 2e stade : acide 4-benzyloxycarbonylméthoxyphénoxyacétique

3,8 g du phénol obtenu au 1er stade sont portés au reflux du THF (200 ml) en présence de 1,3 g de NaH (dispersion à 60 %) et 2 g d'acide bromoacétique pendant 48 h. On verse ensuite le milieu réactionnel sur de la glace et on extrait à l'éther isopropylique puis à l'acétate d'éthyle. La phase aqueuse est acidifiée et extraite par du CH₂Cl₂ pour 2,8 g (Rdt 60 %) d'un solide crème.
¹H 200MHz RMN (CDCl₃) δ 7,35 (5H, s, Ar), 6,78 (4H, s, ArO), 5,15 (2H, s, CH₂ Ar), 4,55 (2H, s, OCH₂ ester), 4,45 (2H, s, OCH₂ acide)

### 3e stade : comptage de l'acide du 2e stade avec le 4'-benzyloxy carbonyl étoposide

A 1,75 g (5,5 mmoles) de l'acide du 2e stade en solution dans 40 ml de CH₂Cl₂ avec 0,2 ml de DMF, on additionne à 0°C sous atmosphère d'azote goutte à goutte 770 mg (6,1 mmoles) de chlorure d'oxalyle. L'agitation est maintenue 2 h à température ambiante. On ajoute alors, après retour à 0°C, une solution de 1 g (1,38 mmoles) de 4'-benzyloxycarbonyl étoposide et de 1,1 g (1,38 mmoles) de pyridine dans 10 ml de CH₂Cl₂. Après 3 h d'agitation à température ambiante, le milieu réactionnel est concentré, repris par l'acétate d'éthyle et lavé à l'eau, puis par une solution glacée de bicarbonate de sodium, après un nouveau lavage par du HCl N, puis par une solution saturée de NaCl, la phase organique est décantée, séchée, évaporée pour fournir 800 mg (Rdt 66 %) qui sont directement hydrogénolysés au stade suivant (CCM SiO₂ heptane-AcOEt 20-80 Rf=0,9)

### 4e stade : Hydrogénolyse

800 mg (0,6 mmoles) de dérivé obtenu au 3e stade sont placés sous atmosphère d'hydrogène à pression atmosphérique dans 15 ml d'acétate d'éthyle et 5 ml d'éthanol en présence de 100 mg de charbon palladié, sous agitation importante pendant une heure. Le catalyseur est filtré et le filtrat évaporé. Le résidu est repris dans l'acétone et filtré à nouveau et évaporé pour fournir quantitativement (650 mg) le dérivé débenzylé. Ce dérivé est transformé en sel de N-méthyl glucamine par addition de 2 équivalents d'une solution 0,1M de N-méthyl glucamine dans le mélange EtOH, H₂O-Acétone. Après 1 h d'agitation, la solution est évaporée, reprise par H₂O, filtrée sur filtre 0,45 µ et lyophylisée on obtient alors 700 mg du dérivé carboxylique.
F ∼ 130°C Anal C₆₃ H₈₂ N₂ O₃₃, 6H₂ O PM = 1503,422

| | C | H | N |
|---|---|---|---|
| Calc % | 50,33 | 6,30 | 1,86 |
| Tr % | 49,89 | 5,93 | 2,19 |

IR ν (KBr) 3427, 1772, 1618, 1508, 1425, 1205, 1087
¹H 200MHz RMN (DMSO) δ 7,04 (1H, s, H₅), 6,59-6,70 (8H, m, OArO), 6,47 (1H, s, H₈), 6,13 (2H, s, H_{2'} et H_{6'}), 5,96 (1H, s, OCH_{A}O), 5,73 (1H, s, OCH_{B}O), 5,33 (2H, m, H_{2"} et H_{3"}), 2,46 (6H, s, NCH₃), 1,20 (3H, d, J=4Hz, H_{8"}).

Par la même suite de réactions de l'exemple 15, mais en utilisant les réactifs appropriés les dérivés de formule générale I (R'=H) sont obtenus :

### Exemple 16 - formule I

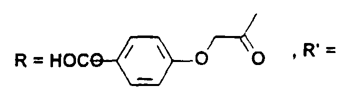

### 4'-déméthyl-4-O-(2,3-bis-(phénylsulfonylacétyl)4.6 Ethylidène-β-D-glucosyl)-épipodophyllotoxine.

Rdt = 92 %
F=248°C Anal. C₄₅ H₄₄ O₁₉ S₂ PM = 976,790

| | C | H |
|---|---|---|
| Calc. % | 56,72 | 4,65 |
| Tr. % | 56,40 | 4,66 |

### Exemple 17 - formule I

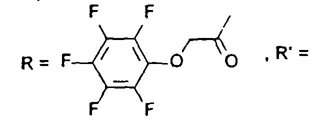

### 4'-déméthyl-4-O-(2,3-bis-(pentafluorophénoxyacétyl4.6 Ethylidène-β-D-glucosyl)-épipodophyllotoxine.

Rdt = 75 % Anal. C₄₅ H₃₄ O₁₇ F₁₀ PM = 1036,75

| | C | H |
|---|---|---|
| Calc. % | 52,13 | 3,30 |
| Tr. % | 51,88 | 3,25 |

### Exemple 18 - formule I

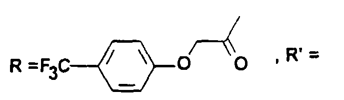

### 4'-déméthyl-4-O-(2,3-bis-(4-trifluorométhylphénoxyacétyl)-β-D-glucosyl)-épipodophyllotoxine.

Rdt = 53 % Anal. C₄₇ H₄₂ O₁₇ F₆ PM = 992,820

| | C | H |
|---|---|---|
| Calc. % | 56,86 | 4,26 |
| Tr. % | 56,78 | 4,22 |

### Exemple 19 - formule I

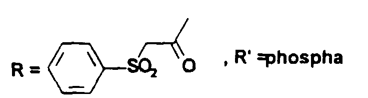

### 4'-déméthyl-4'-désoxy-4'-phosphate-4-O-(2,3-bis-(phénylsulfonylacétyl)-4,6-éthylidène-β-D-glucosyl)-épipodophyllotoxine.

Di-sel de N-méthylglucamine

En utilisant la méthode de l'exemple 1, mais avec le composé de l'exemple 16, le dérivé phosphate sous forme de sel de N-méthylglucamine est obtenu. Rdt = 60 % Anal. C₅₉ H₇₉ N₂ O₃₂ PS₂; 3,8H₂O
F~148°C
PM = 1492,85

| | C | H | N |
|---|---|---|---|
| Calc. % | 49,79 | 5,59 | 1,97 |
| Tr. % | 47,47 | 5,85 | 1,88 |

### Exemple 20 - formule I

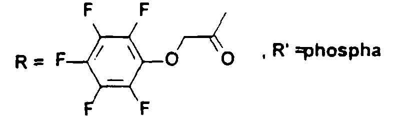

### 4'-déméthyl-4'-désoxy-4'-phosphate-4-O-(2,3-bis-(2,3,4,5,6-pentafluorophénoxyacétyl)-4,6-éthylidène-β-D-glucosyl)-épipodophyllotoxine. Di-sel de N-méthylglucamine.

En utilisant la méthode de l'exemple 1 mais avec le composé de l'exemple 17, le dérivé phosphate sous forme de sel de N-méthylglucamine est obtenu.
Rdt = 78 %
F ~ 140°C Anal. C₅₉ H₆₉ F₁₀ N₂ O₃₀ P; 3,5H₂O PM = 1507,147

| | C | H | N |
|---|---|---|---|
| Calc. % | 45,11 | 4,88 | 1,78 |
| Tr. % | 45,34 | 4,67 | 1,82 |

### Exemple 21 - formule I

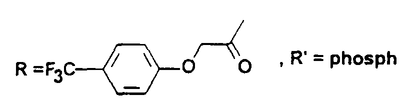

### 4'-déméthyl-4'-désoxy-4'-phosphate-4-O-(2,3-bis-(4-trifluorométhylphénoxyacétyl)-4,6-éthylidène-β-D-glucosyl)-épipodophyllotoxine. Di-sel de N-méthylglucamine.

En utilisant la méthode de l'exemple 1 mais avec le composé obtenu à l'exemple 18, le dérivé phosphate sous forme de N-méthylglucamine est obtenu.
Rdt = 33 %
F = 120°C Anal. C₆₁ H₇₇ N₂ O₃₀ F₆ P; 4,15 H₂O PM = 1538,170

| | C | H | N |
|---|---|---|---|
| Calc. % | 47,63 | 5,59 | 1,82 |
| Tr. % | 47,16 | 5,12 | 1,84 |

### Exemple 22 - formule I

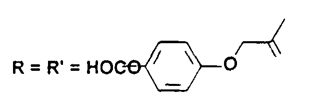

Voie C

### 4'-déméthyl-4'-(4-carboxyphénoxyacétyl)-4-O-(2,3-bis-(4-carboxyphénoxyacétyl)-4,6-éthylidène-β-D-glucosyl)-épipodophyllotoxine- sel de glucamine.

### 1er stade : Condensation de l'acide 4-benzyloxycarbonylphénoxy acétique avec l'étoposide

A une solution de 4,9 g (17 mmoles) d'acide 4-benzyloxycarbonylphénoxyacétique dans 100 ml de CH₂Cl₂ et 0,2 ml de DMF sont ajoutés à 0°C sous azote, goutte à goutte 2,4 g (18,7 mmoles) de chlorure d'oxalyle, après 2 h d'agitation à température ambiante, on introduit à cette solution 2 g (3,4mMoles) d'étoposide en solution dans 15 ml de CH₂Cl₂ et 3,2 g (41 mmoles) de pyridine goutte à goutte à 0°C. Après retour à température ambiante en 3 heures, le milieu réactionnel est versé sur HCl N puis extrait par CH₂Cl₂ et lavé par une solution de NaHCO₃, et NaCl saturé successivement pour obtenir après évaporation un produit brut qui est chromatographié sur SiO₂ par élution dans un mélange heptane-AcOEt (60-40). On obtient 1,8 g (Rdt 38 %) de dérivé trisubstitué qui est utilisé directement dans l'étape suivante.

### 2e stade : Hydrogénolyse

1,5 g (1,07 mmoles) du triester benzylique précédent est hydrogénolysé en présence d'hydrogène à pression atmosphérique dans un mélange EtOH (15 ml) AcOEt (60 ml) avec 300 mg de charbon palladié à 10 % sous bonne agitation pendant 8 h à température ambiante. Le catalyseur est filtré, le filtrat évaporé est chromatographié sur SiO₂ et élué dans un mélange CH₂Cl₂-MeOH (96,4) pour obtenir un solide blanc (600 mg Rdt 50 %). Le sel de glucamine effectué directement, est obtenu en plaçant 3 équivalents d'une solution aqueuse 0,1M de N-méthyl glucamine dans un mélange EtOH-H₂O (80-20), cette solution est ajoutée à la solution du triacide dans l'acétone. Un précipité gommeux est obtenu, le milieu est évaporé puis repris par H₂O et filtré à travers un filtre de 0,45 µ. Le filtrat est alors lyophilisé pour obtenir le sel de glucamine. Rdt = 50 %
F~ 135°C Anal C₇₇ H₁₀₁ N₃ O₄, 7H₂O PM = 1836,103

| | C | H | N |
|---|---|---|---|
| Calc % | 50,37 | 6,32 | 2,29 |
| Tr % | 49,99 | 5,77 | 2,43 |

IR ν (KBr) 3404, 1772, 1604, 1545, 1385, 1086.
¹H 200MHz RMN (DMSO), 7,77-7,85 (6H, m, Ar), 7,1 (1H, s, H₅), 6,89, 6,79, 6,64 (6H, d, J=8,7Hz, ArO), 7,08 (1H, s, H₅), 6,46 (1H, s, H₈), 6,26 (2H, s, H_{2'} et H_{6'}), 6,13 (1H, s, OCH_{A}O), 5,95 (1H, s, OCH_{B}O), 2,42 (9H, s, N-CH₃), 1,20 (3H, d, J=4,9Hz, H_{8"}).

### Exemple 23 - formule I

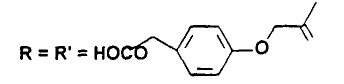

### 4'-déméthyl-4'-carboxyméthylphénoxyacétyl-4-O-(2,3-bis-(4-carboxyméthylphénoxy acétyl)-4,6-éthylidène-β-D-glucosyl)--épipodophyllotoxine.

Ce dérivé est obtenu par la même méthode que pour l'exemple 22, mais en utilisant l'acide benzyloxycarbonylméthylphénoxyacétique. Rdt = 20 %
F~150°C Anal C₅₉ H₅₆ O₂₅, 1.4H₂O PM = 1190,703

| | C | H |
|---|---|---|
| Calc % | 59,63 | 4,81 |
| Tr % | 59,19 | 4,84 |

### Exemple 24 - formule I

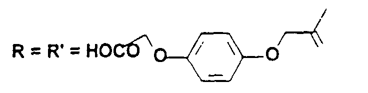

### 4'-déméthyl-4'-carboxyméthoxyphénoxyacétyl-4-O-(2,3-bis-(4-carboxyméthoxy phénoxyacétyl)-4,6-ethylidène-β-D-glucosyl) épipodophyllotoxine - Sel de N-méthyl glucamine.

Ce dérivé est obtenu par la même méthode que pour l'exemple 22, mais en utilisant l'acide benzyloxycarbonylméthoxyphénoxyacétique. Rdt = 84 %
F~110°C Anal C₈₀ H₁₀₇ N₃ O₄₃, 6.3 H₂O PM = 1912,036

| | C | H | N |
|---|---|---|---|
| Calc % | 50,25 | 6,30 | 2,20 |
| Tr % | 50,13 | 6,17 | 2,56 |

### Exemple 25 - formule I

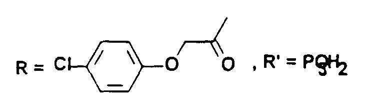

### 4'-déméthyl-4'-désoxy-4'-phosphate-4-O-(2,3-bis-(4-chlorophénoxyacétyl)-4,6-éthylidène-β-D-glucosyl)-épipodophyllotoxine.

### Préparation des intermédiaires (voie B)

4'-déméthyl-4'-(3-quinuclidinylaminocarbonyl)-4-O-(2,3-bis-(4-chlorophénoxy acétyl)-4,6-éthylidène-β-D-glucosyl)-épipodophyllotoxine.

### 1er stade : 4'-(3-quinaclidinylaminocarbonyl) étoposide

formule V R₄ = COHN 3-quinuclidinyl

A une solution 1,93M de phosgène dans l'acétonitrile (8,8 ml, 16,9 mmoles) refroidie à 0°C sous azote, est ajoutée goutte à goutte une solution d'étoposide (5 g, 8,49 mmoles) dans 200 ml de CH₃CN et 2,74 g (21,2 mmoles) de N, N-diisopropyléthylamine en 10 mn, puis introduire 1,07 g (8,49 mmoles) de 3-aminoquinuclidine en solution dans 20 ml de CH₃CN. Le milieu est agité 24 h. Après évaporation, le résidu est chromatographié sur SiO₂ avec un mélange de solvants : CHCl₃-MeOH-NH₄OH (93-7-0,7) puis (90-10-1). On obtient 1,67g (Rdt 26 %) de produit carbamate, V homogène en CCM qui est mis aussitôt en réaction dans le 2e stade.

### 2e stade : Condensation avec l'acide p-chlorophenoxyacétique

A une solution de 1,68 g (8,98 mmoles) d'acide p-chlorophénoxyacétique dans du chloroforme (40 ml) et 0,5 ml de DMF à 0°C sous atmosphère d'azote, sont introduits 1,25 g (9,88 mmoles) de chlorure d'oxalyle goutte à goutte. L'agitation est maintenue 1 h. Cette solution est ensuite ajoutée goutte à goutte à la solution du dérivé de l'étoposide obtenu au 1er stade (1,66 g, 2,24 mmoles) dans 60 ml de chloroforme et 1,77 g de pyridine à 0°C. Cette nouvelle solution est agitée 5 h avec retour à la température ambiante. Le milieu réactionnel est ensuite versé sur HCl N (100 ml) décanté, puis lavé par une solution saturée de NaCl, séchée, évaporée pour fournir une huile qui est chromatographiée sur silice. L'élution par un mélange de CHCl3, MeOH, NH₄OH (95, 5, 0,5) fournit 1,97 g (Rdt 80 %) du dérivé du titre de l'exemple 26. On en forme le chlorhydrate (par addition d'une solution d'éther saturé de gaz chlorhydrique à la solution de la base dans l'acétone. Après agitation pendant 10mn, le précipité obtenu est filtré lentement, lavé à l'éther et séché (Rdt 50 %).
F°~180°C Anal C₅₃ H₅₄ Cl₂ N₂ O₁₈, HCl, 2H₂O PM = 1150,422

| | C | H | N |
|---|---|---|---|
| Calc % | 55,33 | 5,08 | 2,43 |
| Tr % | 55,74 | 4,96 | 2,61 |

Spectre de masse (FAB) m/e 1077 (M⁺)

### 3e stade : hydrolyse

A une solution dans 70 ml d'acétone de 1,08 g du dérivé du 2e stade, on introduit 30 ml d'une solution saturée de NaHCO₃. Le milieu est agité pendant 2 jours, on évapore l'acétone et acidifie le milieu avec HCl concentré jusqu'à pH 2, puis on extrait par du chlorure de méthylène. Une chromatographie sur SiO₂ (élution CH₂Cl₂-MeOH 97-3) fournit le dérivé du titre de l'exemple 27. Une nouvelle chromatographie sur SiO₂ (élution éther de pétrole - AcOEt 1-1) fournit après évaporation un résidu qui cristallise dans l'éther isopropylique (200mg Rdt=21 %).
F~125°C Anal C₄₅ H₄₂ Cl₂ O₁₇ PM = 925,730

| | C | H |
|---|---|---|
| Calc % | 58,38 | 4,57 |
| Tr % | 58,63 | 4,69 |

Spectre de masse (FAB) m/e 924 (M⁺-1)
IR v (KBr) 3458, 1774, 1618, 1491.
¹H 200MHz RMN (CDCl₃) δ 7,15-7,22 (4H, m, Ar), 6,75 (1H, s, H₅), 6,77 (2H, d, J=8,8Hz, ArO), 6,64 (2H, d, J=8,8Hz, ArO), 6,51 (1H, s, H₈), 6,22 (2H, s, H_{2'} et H_{6'}), 5,91 (1H, s, OCH_{A}O); 5,71 (1H, s, OCH_{B}O), 5,33 (1H, t, J=9Hz, H_{3"}), 5,02 (1H, t, J=7,8Hz, H_{2"}), 4,91 (1H, d, J=7,8Hz, H_{1"}), 4,83 (1H, d, J=3,2Hz, H₄), 4,69 (1H, q, H_{7"}), 3,1 (1H, dd, H₂), 2,9 (1H, m, H₃), 1,34 (3H, d, J=5Hz, H_{8"}).

### Préparation du phosphate :

Ce dérivé est obtenu selon le mode opératoire de l'exemple 1 mais en utilisant le dérivé obtenu au 3e stade.

### Sel de N-méthylglucamine:

Rdt = 70 %
F~140°C Anal C₅₉ H₇₇ Cl₂ N₂ O₃₀ P, 6H₂O PM = 1461,49

| | C | H | N |
|---|---|---|---|
| Calc % | 48,49 | 5,8 | 1,90 |
| Tr % | 48,78 | 5,55 | 1,88 |

### Exemple 26 - formule I

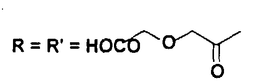

### 4'-déméthyl-4'-carboxyméthoxyacétyl-4-O-(2,3-bis-carboxyméthoxyacétyl-4,6-éthylidène-β-D-glucosyl) épipodophyllotoxine.

Ce dérivé est préparé selon la méthode de l'exemple 22, 1er et 2e stade dans lequel on utilise l'acide benzyloxycarbonylméthoxy acétique au lieu de l'acide 4-benzyloxycarbonylphénoxyacétique, pour conduire avec des rendements successifs de 77 % et de 75 %.
F=138°C Anal C₄₁ H₄₄ O₂₅, H₂O PM = 954,805

| | C | H |
|---|---|---|
| Calc % | 51,57 | 4,85 |
| Tr % | 51,22 | 4,72 |

Spectre de masse (FAB) m/e 959 (M⁺ +Na)

### Exemple 27 - formule I

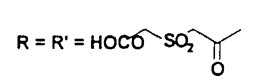

### 4'-déméthyl-4'-carboxyméthylsulfonylacétyl-4-O-(2,3-bis-carboxyméthylsulfonyl acétyl-4,6-éthylidène-β-D-glucosyl) épipodophyllotoxine.

Ce dérivé est préparé comme le précédent selon la méthode de l'exemple 22, 1er et 2e stades en utilisant l'acide benzyloxycarbonylméthylsulfonylacétique avec des rendements successifs de 55 % et 90 %.
F~165°C Anal C₄₁ H₄₄ O₂₈ S₃, H₂O PM = 1098,98

| | C | H |
|---|---|---|
| Calc % | 44,81 | 4,22 |
| Tr % | 44,94 | 4,34 |

Spectre de masse (FAB) m/e 1103 (M⁺ +Na)

### Exemple 28 - formule I

R = HOCOC₂HOCH₂CO, R' =

### 4'-déméthyl-4-O-(2,3-bis-carboxyméthoxyacétyl-4,6-éthylidène-β-D-glucosyl) épipodophyllotoxine.

Ce dérivé est obtenu par la méthode décrite pour l'exemple 15, 3e et 4e stades, mais en utilisant l'acide benzyloxycarbonylméthoxyacétique au lieu de benzyloxycarbonylméthoxyphénoxyacétique. Les rendements successifs sont 42 % et 71 %.
F~192°C Anal C₃₇ H₄₀ O₂₁, 0.2 H₂O PM = 824,32

| | C | H |
|---|---|---|
| Calc % | 54,59 | 5,20 |
| Tr % | 54,81 | 5,23 |

### Exemple 29 - formule I

R = R' =H₂PO₃CH₂C

### 4'-déméthyl-4'-Phosphonoacétyl-4-O-(2,3-bis-phosphonoacétyl-4,6-éthylidène-β-D-glucosyl) épipodophyllotoxine.

Ce dérivé est obtenu de façon identique à la méthode de l'exemple 22, 1er et 2e stades, mais en utilisant l'acide dibenzylphosphonoacétique (Tet. Let. 1974, N°9, 711). Les modifications par rapport à l'exemple 22 sont les suivants : le premier stade se conduit à 0°C avec retour à température ambiante pendant 18 h ; l'hydrogénolyse au second stade se fait dans le solvant : THF 25 ml et EtOH 50 ml et la réaction est conduite à 0°C pendant 4h. Les rendements sont successivement 34 % et 83 %.
F~184°C Anal C₃₅ H₄₁ O₂₅ P₃, 4H₂O PM = 1026, 58

| | C | H | H2O |
|---|---|---|---|
| Calc % | 40,94 | 4,81 | 7,02 |
| Tr % | 40,95 | 4,38 | 7,54 |

Sprectre de masse (FAB) m/e 955 (M⁺ +1)

### Exemple 30 - formule I

R = H₂O₃PCH₂CO, R' =

### 4'-déméthyl-4-O-(2,3-bis-phosphonoacétyl-4,6-éthylidène-β-D-glucosyl) épipodophyllotoxine.

Ce dérivé est obtenu de façon identique à la méthode de l'exemple 15, 3e et 4e stades, mais en utilisant l'acide dibenzylphosphonoacétique, déjà utilisé pour l'exemple 29.

Le stade d'hydrogénolyse se fait en utilisant un mélange de solvant : THF-EtOH (30-70) à 0°C pendant 4h. On obtient alors le dérivé avec un rendement global de 45 %.
F~168°C Anal C₃₃ H₃₈ O₂₁ P₂ PM = 832,606
Spectre de masse (FAB) m/e 833 (M⁺ +1).

Les composés suivants selon l'invention ont également été préparés :

### 4'-déméthyl-4'-désoxy, 4'-phosphate-4-O-(2,3-bis(4-cyanophénoxyacétyl)-4,6-éthylidène-β-D-glucosyl) épipodophyllotoxine. Sel de N-méthylglucamine.

F~205°C Anal C₄₇ H₄₃ N₂ O₂₀ P, 6 H₂O = 1 112,93

| | C | H | N |
|---|---|---|---|
| Calc % | 51,56 | 5,06 | 2,56 |
| Tr % | 51,11 | 4,27 | 2,31 |

### 4'-déméthyl-4'-désoxy, 4'-phosphate-4-O-(2,3-bis(4-nitrophénoxyacétyl)-4,6-éthylidène-β-D-glucosyl) épipodophyllotoxine. Sel de N-méthylglucamine.

F~190°C Anal C₄₅ H₄₃ N₂ O₂₄ P, 4 H₂O = 1 098,88

| | C | H | N |
|---|---|---|---|
| Calc % | 49,18 | 4,68 | 2,55 |
| Tr % | 49,38 | 4,25 | 2,46 |

### 4'-déméthyl-4'-désoxy, 4'-phosphate-4-O-(2,3-bis(4-méthylphénoxyacétyl)-4,6-éthylidène-β-D-glucosyl) épipodophyllotoxine.

F~190°C Anal C₄₇ H₄₉ N₂ O₂₀ P, 1,25 H₂O = 987,378

| | C | H |
|---|---|---|
| Calc % | 57,12 | 5,21 |
| Tr % | 56,6 | 5,04 |

### 4'-déméthyl-4'-désoxy-4'-phosphate-4-O-(2,3-bis(4-hydroxyphénoxyacétyl)-4,6-éthylidène-β-D-glucosyl) épipodophyllotoxine. Sel de N-méthylglucamine.

F~145°C Anal C₅₉ H₇₉ N₂ O₃₂ P, H₂O = 1 377,26

| | C | H | N |
|---|---|---|---|
| Calc % | 51,45 | 5,93 | 2,03 |
| Tr % | 51,44 | 6,17 | 2,04 |

## Revendications

1. Composé de formule générale **I** 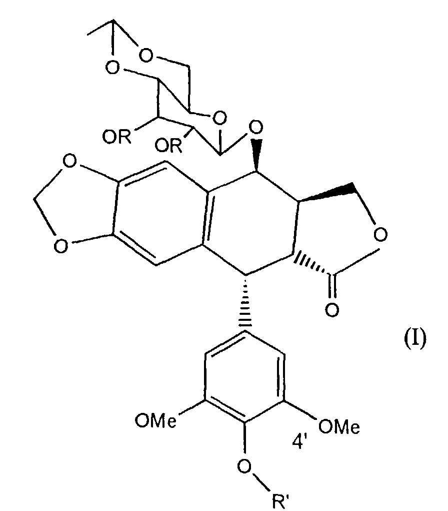 dans laquelle R' représente un groupement phosphate monoester ; un groupement carbamate du type -CO-N(R₁R₂) où N(R₁R₂) représente un groupement aminodiacétique ou une amine polycyclique comme la 3-amino-quinuclidine; un groupement phosphonoacétique CO-CH₂-PO₃H₂ ; CO-CH₂-SO₂CH₂COOH; CO-CH₂-OCH₂COOH; ou un radical R de formule A-Z-CH₂-CO dans laquelle
• Z représente un atome d'oxygène, de soufre, un groupement SO₂, un alkylène linéaire ou ramifié en C₁₋₄, et
• A représente un noyau phényl substitué ou non à la condition que :
- dans le cas où R' = R, c'est-à-dire les dérivés triacylés, A représente un noyau aromatique possédant une fonction salifiable, sauf 4-hydroxy-phényle,
- dans le cas où R' ≠ R, A représente un reste benzyl, naphtyl, hétéroaryl, phényl substitué ou non, dans ce cas le phényl pouvant être substitué une, deux, trois, quatre ou cinq fois quelle que soit sa position sur le noyau aromatique par des groupes identiques ou différents choisis parmi les groupes halogènes, F, Cl, Br, alcoxy linéaire ou cycliques en C₁₋₆, alkyl C₁-C₆, méthylène dioxy, OCF₃, CF₃, NO₂, CN, OCH₂ Aryl, OH, OPO₃H₂, CH₂-PO₃H₂, PO₃H₂, OCH₂CO₂H, COOH, CH₂COOH, COCH₃, CHO,
ainsi que ses sels avec des acides ou des bases minérales ou organiques, thérapeutiquement acceptables et hydrosolubles.

2. Composé selon la revendication 1, caractérisé en ce que R' représente un groupement phosphate monoester (PO₃H₂) ; un carbamate CONR₁R₂ où NR₁R₂ représente un groupement aminodiacétique ou une amino-3-quinuclidine ; ou CO-CH₂-PO₃H₂.

3. Composé selon les revendications 1 et 2, caractérisé en ce que R est choisi parmi les radicaux :
phénoxyacétyl, 3,4-méthylènedioxyphénoxyacétyl,
4-méthoxyphénoxyacétyl, 4-hydroxyphénoxyacétyl,
4-phosphonooxyphénoxyacétyl, 4-carboxyméthylphénoxyacétyl,
4-carboxyméthoxyphénoxyacétyl, 4-carboxyphénoxyacétyl,
4- trifluorométhylphénoxyacétyl, 4- trifluorométhoxyphénoxyacétyl,
4-chlorophénoxyacétyl, 4-nitrophénoxyacétyl,
4-fluorophénoxyacétyl, cyclohexyloxyacétyl, phénylsulfonylacétyl, pentafluorophénoxyacétyl, 2 et 4-formylphénoxyacétyl,
4-cyanophénoxyacétyl.

4. Composé selon l'une des revendications 1 à 3, caractérisé en ce qu'il est choisi parmi les sels organiques ou minéraux de :
• 4'-déméthyl-4'-deoxy-4'-phosphate-4-O-(2,3-bis phénoxyacétyl-4,6-éthylidène-β-D-glucosyl)-épipodophyllotoxine,
• 4'-déméthyl-4'-di(carboxyméthyl)aminocarbonyl-4-O-(2,3-bis phénoxyacétyl-4,6-éthylidène-β-D-glucosyl)-épipodophyllotoxine,
• 4'-déméthyl-4'-phosphonoacétyl-4-O-(2,3-bis phénoxyacétyl-4,6-éthylidène-β-D-glucosyl)-épipodophyllotoxine,
• 4'-déméthyl-4'-désoxy-4'-phosphate-4-O-(2,3-bis-(4-trifluorométhylphénoxyacétyl)-4,6-éthylidène-β-D-glucosyl)-épipodophyllotoxine,
• 4'-déméthyl-4'-di(carboxyméthyl)aminocarbonyl-4-O-(2,3-bis-(4-trifluorométhoxyphénoxyacétyl)-4,6-éthylidène-β-D-glucosyl)-épipodophyllotoxine,
• 4'-déméthyl-4'-désoxy-4'-phosphate-4-O-(2,3-bis-(4-trifluorométhoxyphénoxyacétyl)-4,6-éthylidène-β-D-glucosyl)-épipodophyllotoxine,
• 4'-déméthyl-4'-phosphonoacétyl-4-O-(2,3-bis-(4-trifluorométhoxyphénoxyacétyl)-4,6-éthylidène-β-D-glucosyl)-épipodophyllotoxine,
• 4'-déméthyl-4'-(4-phosphonooxyphénoxyacétyl)-4-O-(2,3-bis-(4-phosphonooxyphénoxyacétyl)-4,6-éthylidène-β-D-glucosyl)-épipodophyllotoxine,
• 4'-déméthyl-4'-désoxy-4'-phosphate-4-O-(2,3-bis-(4-phosphonooxyphénoxyacétyl)-4,6-éthylidène-β-D-glucosyl)-épipodophyllotoxine,
• 4'-déméthyl-4'-di(carboxyméthyl)aminocarbonyl-4-O-(2,3-bis-cyclohexyloxyacétyl-4,6-éthylidène-β-D-glucosyl)-épipodophyllotoxine,
• 4'-déméthyl-4'-désoxy-4'-phosphate-4-O-(2,3-bis cyclohexyloxyacétyl)-4,6-éthylidène-β-D-glucosyl)-épipodophyllotoxine,
• 4'-déméthyl-4'-(3-quinuclidinyl aminocarbonyl)-4-O-(2,3-bis-(3,4-méthylènedioxyphénoxyacétyl)-4,6-éthylidène-β-D-glucosyl)-épipodophyllotoxine,
• 4'-déméthyl-4'-désoxy-4'-phosphate-4-O-(2,3-bis-(3,4-méthylènedioxyphénoxyacétyl)-4,6-éthylidène-β-D-glucosyl)-épipodophyllotoxine,
• 4'-déméthyl-4'-désoxy-4'-phosphate-4-O-(2,3-bis-(2,3,4,5,6-pentafluorophénoxyacétyl)-4,6-éthylidène-β-D-glucosyl)-épipodophyllotoxine,
• 4'-déméthyl-4'-désoxy-4'-phosphate-4-O-(2,3-bis-(4-fluorophénoxyacétyl)-4,6-éthylidène-β-D-glucosyl)-épipodophyllotoxine,
• 4'-déméthyl-4'-(3-quinuclidinylaminocarbonyl)-4-O-(2,3-bis-(4-chlorophénoxyacétyl)-4,6-éthylidène-β-D-glucosyl)-épipodophyllotoxine,
• 4'-déméthyl-4'-désoxy-4'-phosphate-4-O-(2,3-bis-(4-chlorophénoxyacétyl)-4,6-éthylidène-β-D-glucosyl)-épipodophyllotoxine,
• 4'-déméthyl-4'-désoxy-4'-phosphate-4-O-(2,3-bis-phénylsulfonylacétyl)-4,6-éthylidène-β-D-glucosyl)-épipodophyllotoxine,
• 4'-déméthyl-4'-(4-carboxyphénoxyacétyl)-4-O-(2,3-bis-(4-carboxyphénoxyacétyl)-4,6-éthylidène-β-D-glucosyl)-épipodophyllotoxine,
• 4'-déméthyl-4'-(4-carboxyméthylphénoxyacétyl)-4-O-(2,3-bis-(4-carboxyméthylphénoxyacétyl)-4,6-éthylidène-β-D-glucosyl)épipodophyllotoxine,
• 4'-déméthyl-4'-(4-carboxyméthoxyphénoxyacétyl)-4-O-(2,3-bis-(4-carboxyméthoxyphénoxyacétyl)-4,6-éthylidène-β-D-glucosyl)-épipodophyllotoxine,
• 4'-déméthyl-4'-(3-quinuclidinylaminocarbonyl)-4-O-(2,3-bis-(4-nitrophénoxyacétyl)-4,6-éthylidène-β-D-glucosyl)-épipodophyllotoxine,
• 4'-déméthyl-4'-désoxy-4'-phosphate-4-O-(2,3-bis-(4-méthoxyphénoxyacétyl)-4,6-éthylidène-β-D-glucosyl)-épipodophyllotoxine,
• 4'-déméthyl-4'-désoxy-4'-phosphate-4-O-(2,3-bis(4-cyanophénoxyacétyl)-4,6-éthylidène-β-D-glucosyl)-épipodophyllotoxine,
• 4'-déméthyl-4'-désoxy-4'-phosphate-4-O-(2,3-bis(4-nitrophénoxyacétyl)-4,6-éthylidène-β-D-glucosyl)-épipodophyllotoxine,
• 4'-déméthyl-4'-désoxy-4'-phosphate-4-O-(2,3-bis(4-méthylphénoxyacétyl)-4,6-éthylidène-β-D-glucosyl)-épipodophyllotoxine,
• 4'-déméthyl-4'-désoxy-4'-phosphate-4-O-(2,3-bis(4-hydroxyphénoxyacétyl)-4,6-éthylidène-β-D-glucosyl)-épipodophyllotoxine.

5. Composés selon la revendication 4, caractérisés en ce que les agents salifiant les fonctions acides sont plus particulièrement choisis parmi les amines telles que N-méthylglucamine, lysine, triéthanolamine, ou les cations comme le sodium ou le potassium et en ce que les agents salifiant les fonctions basiques sont plus particulièrement choisis parmi les acides minéraux ou organiques tels que acide chlorhydrique, acide sulfurique, acide méthane sulfonique, acide éthanol sulfonique, acide maléique, acide tartrique.

6. Procédé de préparation d'un composé selon l'une des revendications 1 à 4, caractérisé en ce que l'on fait réagir un intermédiaire glycosylé de formule générale **II**. 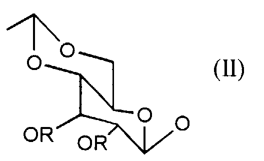 avec un intermédiaire **III** 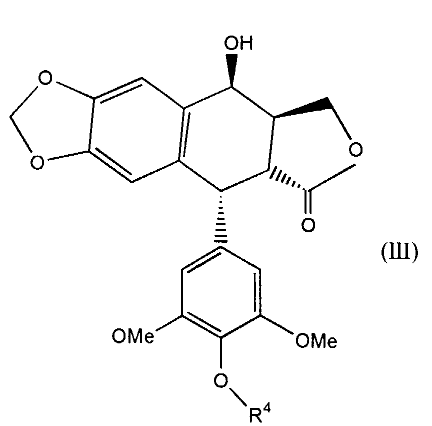 pour lesquels R est défini comme dans la revendication 1 et R⁴ est un groupe protecteur, par exemple le benzyloxycarbonyl ou un reste carbamate que l'on déprotège pour obtenir les composés de formule I R' = H, et que l'on fait réagir avec les réactifs appropriés pour obtenir les composés de formule I R' ≠ H.

7. Procédé de préparation d'un composé selon l'une des revendications 1 à 4, caractérisé en ce que l'on fait réagir l'étoposide protégé en position 4' par un groupe benzyloxycarbonyl ou quinuclidinecarbamate de formule V dans laquelle R⁴ est défini comme dans la revendication 6 avec un réactif acylant de type A-Z-CH₂CO pour conduire après 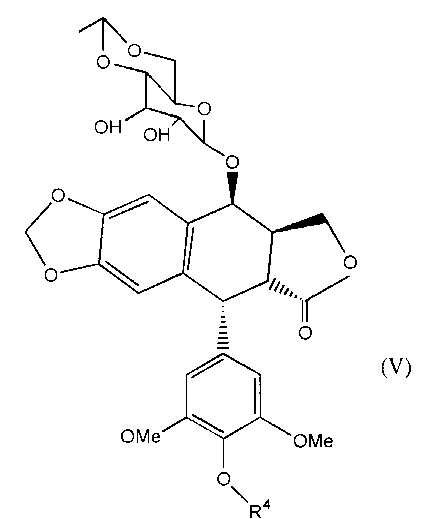 hydrogénolyse ou hydrolyse au composé de formule I tel que R' = H.

8. Procédé de préparation d'un composé selon l'une des revendications 1 à 4, caractérisé en ce que l'on fait réagir l'étoposide par triacylation pour obtenir les composés de formule I où R' = R = A-Z-CH₂CO. A possédant une fonction salifiable.

9. Procédé de préparation du sel d'un composé selon les revendications 1 à 5, caractérisé en ce que l'on traite le composé acide par une quantité stoechiométrique de base, ou par résine échangeuse d'ions et que l'on lyophilise ou cristallise.

10. Procédé de préparation du sel d'un composé selon les revendications 1 à 5, caractérisé en ce que l'on traite le composé basique par une quantité stoechiométrique de l'agent acide et que l'on lyophilise ou cristallise.

11. A titre de médicament, le composé selon l'une des revendications 1 à 5.

12. Composition pharmaceutique caractérisée en ce qu'elle comprend au moins un composé de formule I selon l'une des revendications 1 à 5 et un excipient approprié.

13. Utilisation d'un composé de formule I selon l'une des revendications 1 à 5, pour la préparation d'un médicament destiné au traitement anticancéreux, en particulier le cancer du poumon à petites cellules, les tumeurs embryonnaires, les neuroblastomes, le cancer du rein, les tumeurs pédiatriques, les lymphomes hodgkimiens et non hodgkimiens, les leucémies aigües, les choriocarcinomes placentaires, les adenocarcinomes mammaires ainsi que pour augmenter l'efficacité thérapeutique des composés inhibiteurs de topoisomérase Il pour le traitement des tumeurs réfractaires aux thérapeutiques usuelles : les cancers colorectaux et les mélanomes.

14. Utilisation d'un composé de formule I selon l'une des revendications 1 à 5, pour la préparation d'un médicament destiné au traitement de l'arthrite rhumatoïde, et des affections provoquées par le papilloma virus humain.

## Patentansprüche

1. Verbindung der allgemeinen Formel I 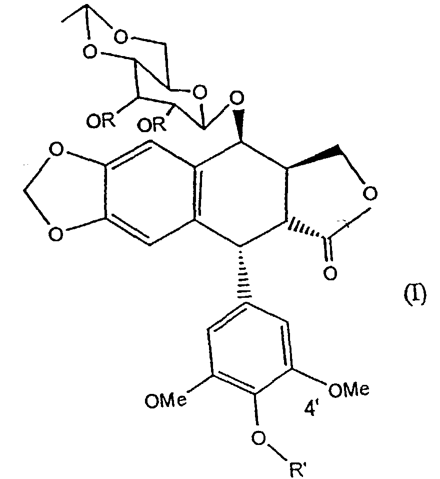 wobei R' eine Phosphatmonoester-Gruppe; eine Carbamat-Gruppe vom Typ -CO-N(R₁R₂), wobei N(R₁R₂) eine Aminodiacetatgruppe oder ein polycyclisches Amin wie 3-Aminochinuclidin darstellt; eine Phosphonoacetatgruppe CO-CH₂-PO₃H₂; CO-CH₂-SO₂CH₂COOH; CO-CH₂-OCH₂COOH; oder einen Rest R der Formel A-Z-CH₂-CO darstellt, wobei
• Z ein Sauerstoff-, Schwefelatom, eine SO₂-Gruppe, eine lineare oder verzweigte (C₁-C₄)Alkylengruppe darstellt, und
• A einen substituierten oder unsubstituierten Phenylkern darstellt, unter der Bedingung daß:
- für den Fall, daß R' = R, d.h. triacylierte Derivate, A einen aromatischen Kern darstellt, der eine versalzbare Funktion aufweist, mit Ausnahme von 4-Hydroxy-phenyl,
- für den Fall, daß R' ≠ R, A einen Benzyl-, Naphthyl-, Heteroaryl-, substituierten oder unsubstituierten Phenylrest darstellt, wobei in diesem Fall Phenyl je nach seiner Position am aromatischen Ring einmal, zweimal, dreimal, viermal oder fünfmal durch gleiche oder verschiedene Gruppen substituiert sein kann, die ausgewählt werden aus den Gruppen Halogen, F, Cl, Br, lineares oder cyclisches (C₁-C₆)Alkoxy, (C₁-C₆)Alkyl, Methylendioxy, OCF₃, CF₃, NO₂, CN, OCH₂-Aryl, OH, OPO₃H₂, CH₂-PO₃H₂, PO₃H₂, OCH₂CO₂H, COOH, CH₂COOH, COCH₃, CHO,
genauso wie ihre therapeutisch verträglichen und wasserlöslichen Salze mit Mineralsäuren oder -basen oder organischen Säuren oder Basen.

2. Verbindung nach Anspruch 1, dadurch charakterisiert, daß R' eine Phosphatmonoester-Gruppe (PO₃H₂); ein Carbamat CONR₁R₂, wobei NR₁R₂ eine Aminodiacetatgruppe oder ein Amino-3-chinuclidin darstellt; oder CO-CH₂-PO₃H₂ darstellt.

3. Verbindung nach den Ansprüchen 1 und 2, dadurch charakterisiert, daß R ausgewählt wird aus den Resten: Phenoxyacetyl, 3,4-Methylendioxyphenoxyacetyl, 4-Methoxyphenoxyacetyl, 4-Hydroxyphenoxyacetyl, 4-Phosphonooxyphenoxyacetyl, 4-Carboxymethylphenoxyacetyl, 4-Carboxymethoxyphenoxyacetyl, 4-Carboxyphenoxyacetyl, 4-Trifluoromethylphenoxyacetyl, 4-Trifluoromethoxyphenoxyacetyl, 4-Chlorophenoxyacetyl, 4-Nitrophenoxyacetyl, 4-Fluorophenoxyacetyl, Cyclohexyloxyacetyl, Phenylsulfonylacetyl, Pentafluorophenoxyacetyl, 2- und 4-Formylyphenoxyacetyl, 4-Cyanophenoxyacetyl.

4. Verbindung nach einem der Ansprüche 1 bis 3, dadurch charakterisiert, daß sie ausgewählt wird aus den organischen Salzen oder Mineralsalzen von:
• 4'-Demethyl-4'-deoxy-4'-phosphat-4-O-(2,3-bisphenoxyacetyl-4,6-ethyliden-β-D-glucosyl)-epipodophyllotoxin,
• 4'-Demethyl-4'-di(carboxymethyl)aminocarbonyl-4-O-(2,3-bisphenoxyacetyl-4,6-ethyliden-β-D-glucosyl)-epipodophyllotoxin,
• 4'-Demethyl-4'-phosphonoacetyl-4-O-(2,3-bis-phenoxyacetyl-4,6-ethyliden-β-D-glucosyl)-epipodophyllotoxin,
• 4'-Demethyl-4'-desoxy-4'-phosphat-4-O-(2,3-bis-(4-trifluoromethylphenoxyacetyl)-4,6-ethyliden-β-D-glucosyl)-epipodophyllotoxin,
• 4'-Demethyl-4'-di(carboxymethyl)aminocarbonyl-4-O-(2,3-bis-(4-trifluoromethoxyphenoxyacetyl)-4,6-ethyliden-β-D-glucosyl)-epipodophyllotoxin,
• 4'-Demethyl-4'-desoxy-4'-phosphat-4-O-(2,3-bis-(4-trifluoromethoxyphenoxyacetyl)-4,6-ethyliden-β-D-glucosyl)-epipodophyllotoxin,
• 4'-Demethyl-4'-phosphonoacetyl-4-O-(2,3-bis-(4-trifluoromethoxyphenoxyacetyl)-4,6-ethyliden-β-D-glucosyl)-epipodophyllotoxin,
• 4'-Demethyl-4'-(4-phosphonooxyphenoxyacetyl)-4-O-(2,3-bis-(4-phosphonooxyphenoxyacetyl)-4,6-ethyliden-β-D-glucosyl)-epipodophyllotoxin,
• 4'-Demethyl-4'-desoxy-4'-phosphat-4-O-(2,3-bis-(4-phosphonooxyphenoxyacetyl)-4,6-ethyliden-β-D-glucosyl)-epipodophyllotoxin,
• 4'-Demethyl-4'-di(carboxymethyl)aminocarbonyl-4-O-(2,3-bis-cyclohexyloxyacetyl-4,6-ethyliden-β-D-glucosyl)-epipodophyllotoxin,
• 4'-Demethyl-4'-desoxy-4'-phosphat-4-O-(2,3-bis-cyclohexyloxyacetyl)-4,6-ethyliden-β-D-glucosyl)-epipodophyllotoxin,
• 4'-Demethyl-4'-(3-chinuclidinylaminocarbonyl)-4-O-(2,3-bis-(3,4-methylendioxyphenoxyacetyl)-4,6-ethyliden-β-D-glucosyl)-epipodophyllotoxin,
• 4'-Demethyl-4'-desoxy-4'-phosphat-4-O-(2,3-bis-(3,4-methylendioxyphenoxyacetyl)-4,6-ethyliden-β-D-glucosyl)-cpipodophyllotoxin,
• 4'-Demethyl-4'-desoxy-4'-phosphat-4-O-(2,3-bis-(2,3,4,5,6-pentafluorophenoxyacetyl)-4,6-ethyliden-β-D-glucosyl)-epipodophyllotoxin,
• 4'-Demethyl-4'-desoxy-4'-phosphat-4-O-(2,3-bis-(4-fluorophenoxyacetyl)-4,6-ethyliden-β-D-glucosyl)-epipodophyllotoxin,
• 4'-Demethyl-4'-(3-chinuchlidinylaminocarbonyl)-4-O-(2,3-bis-(4-chlorophenoxyacetyl)-4,6-ethyliden-β-D-glucosyl)-epipodophyllotoxin,
• 4'-Demethyl-4'-desoxy-4'-phosphat-4-O-(2,3-bis-(4-chlorophenoxyacetyl)-4,6-ethyliden-β-D-glucosyl)-epipodophyllotoxin,
• 4'-Demethyl-4'-desoxy-4'-phosphat-4-O-(2,3-bis-phenylsulfonylacetyl)-4,6-ethyliden-β-D-glucosyl)-epipodophyllotoxin,
• 4'-Demethyl-4'-(4-carboxyphenoxyacetyl)-4-O-(2,3-bis-(4-carboxyphenoxyacetyl)-4,6-ethyliden-β-D-glucosyl)-epipodophyllotoxin,
• 4'-Demethyl-4'-(4-carboxymethylphenoxyacetyl)-4-O-(2,3-bis-(4-carboxymethylphenoxyacetyl)-4,6-ethyliden-β-D-glucosyl)-epipodophyllotoxin,
• 4'-Demethyl-4'-(4-carboxymethoxyphenoxyacetyl)-4-O-(2,3-bis-(4-carboxymethoxyphenoxyacetyl)-4,6-ethyliden-β-D-glucosyl)-epipodophyllotoxin,
• 4'-Demethyl-4'-(3-chinuclidinylaminocarbonyl)-4-O-(2,3-bis-(4-nitrophenoxyacetyl)-4,6-ethyliden-β-D-glucosyl)-epipodophyllotoxin,
• 4'-Demethyl-4'-desoxy-4'-phosphat-4-O-(2,3-bis-(4-methoxyphenoxyacetyl)-4,6-ethyliden-β-D-glucosyl)-epipodophyllotoxin,
• 4'-Demethyl-4'-desoxy-4'-phosphat-4-O-(2,3-bis-(4-cyanophenoxyacetyl)-4,6-ethyliden-β-D-glucosyl)-epipodophyllotoxin,
• 4'-Demethyl-4'-desoxy-4'-phosphat-4-O-(2,3-bis(4-nitrophenoxyacetyl)-4,6-ethyliden-β-D-glucosyl)-epipodophyllotoxin,
• 4'-Demethyl-4'-desoxy-4'-phosphat-4-O-(2,3-bis(4-methylphenoxyacetyl)-4,6-ethyliden-β-D-glucosyl)-epipodophyllotoxin,
• 4'-Demethyl-4'-desoxy-4'-phosphat-4-O-(2,3-bis(4-hydroxyphenoxyacetyl)-4,6-ethyliden-β-D-glucosyl)-epipodophyllotoxin.

5. Verbindungen nach Anspruch 4, dadurch charakterisiert, daß die mit den Säurefunktionen Salze bildenden Mittel insbesondere ausgewählt werden aus Aminen wie N-Methylglucamin, Lysin, Triethanolamin, oder aus Kationen wie Natrium oder Kalium und dadurch, daß die mit den basischen Funktionen Salze bildenden Mittel insbesondere ausgewählt werden aus Mineralsäuren oder organischen Säuren wie Salzsäure, Schwefelsäure, Methansulfonsäure, Ethanolsulfonsäure, Maleinsäure, Weinsäure.

6. Verfahren zur Herstellung einer Verbindung nach einem der Ansprüche 1 bis 4, dadurch charakterisiert, daß man ein glycosyliertes Zwischenprodukt der allgemeinen Formel II 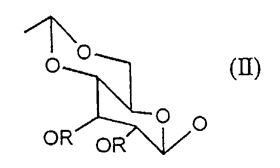 mit einem Zwischenprodukt III 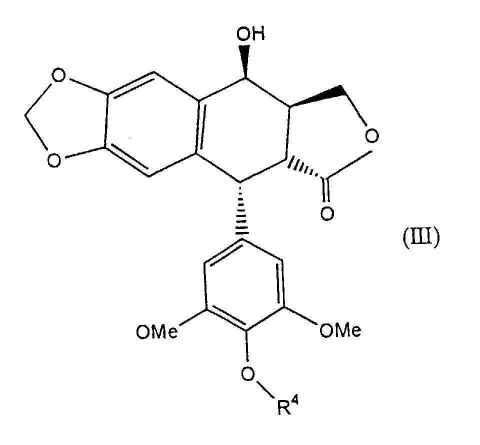 umsetzt, bei denen R wie in Anspruch 1 definiert ist und R⁴ eine Schutzgruppe ist, beispielsweise Benzyloxycarbonyl oder ein Carbamatrest, die man zur Herstellung von Verbindungen der Formel I R' = H entschützt, und die man mit geeigneten Reagentien zur Herstellung von Verbindungen der Formel I R' ≠ H umsetzt.

7. Verfahren zur Herstellung einer Verbindung nach einem der Ansprüche 1 bis 4, dadurch charakterisiert, daß man das in Position 4' mit einer Benzyloxycarbonyl- oder Chinuclidincarbamatgruppe geschütztes Etoposid der Formel V, 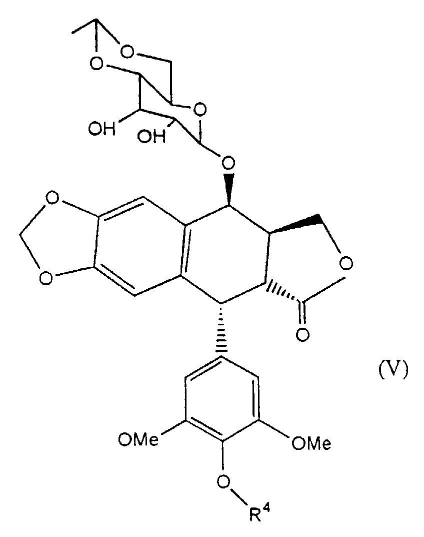 wobei R⁴ wie in Anspruch 6 definiert ist, mit einem Acylierungsmittel vom Typ A-Z-CH₂CO umsetzt, um nach Hydrogenolyse oder Hydrolyse die Verbindung der Formel I zu erhalten, bei der R' = H ist.

8. Verfahren zur Herstellung einer Verbindung nach einem der Ansprüche 1 bis 4, dadurch charakterisiert, daß man Etoposid triacyliert zur Herstellung von Verbindungen der Formel I, bei denen R' = R = A-Z-CH₂CO, wobei A eine versalzbare Funktion aufweist.

9. Verfahren zur Herstellung eines Salzes einer Verbindung nach den Ansprüchen 1 bis 5, dadurch charakterisiert, daß man die saure Verbindung mit einer stöchiometrischen Menge Base oder mit Ionenaustauscherharz behandelt und sie lyophilisiert oder kristallisiert.

10. Verfahren zur Herstellung eines Salzes einer Verbindung nach den Ansprüchen 1 bis 5, dadurch charakterisiert, daß man die basische Verbindung mit einer stöchiometrischen Menge des sauren Mittels behandelt und sie lyophilisiert oder kristallisiert.

11. Verbindung nach einem der Ansprüche 1 bis 5 als Medikament.

12. Pharmazeutische Zusammensetzung, dadurch charakterisiert, daß sie mindestens eine Verbindung der Formel I nach einem der Ansprüche 1 bis 5 und einen geeigneten Exzipienten umfaßt.

13. Verwendung einer Verbindung der Formel I nach einem der Ansprüche 1 bis 5 zur Herstellung eines Medikaments zur Antikrebs-Behandlung, insbesondere von kleinzelligem Lungenkrebs, embryonalen Tumoren, Neuroblastomen, Nierenkrebs, pädiatrischen Tumoren, Hodgkin- und Non-Hodgkin-Lymphomen, akuten Leukämien, Plazenta-Choriokarzinomen, Mamma-Adenokarzinomen, und zur Erhöhung der therapeutischen Wirksamkeit von Topoisomerase II-inhibitorischen Verbindungen für die Behandlung von gegen übliche Therapeutika resistenten Tumoren: kolorektalen Krebsarten und Melanomen.

14. Verwendung einer Verbindung der Formel I nach einem der Ansprüche 1 bis 5 zur Herstellung eines Medikaments zur Behandlung von rheumatoider Arthritis und durch Human-Papilloma-Virus hervorgerufenen Krankheiten.

## Claims

1. Compound of general formula I 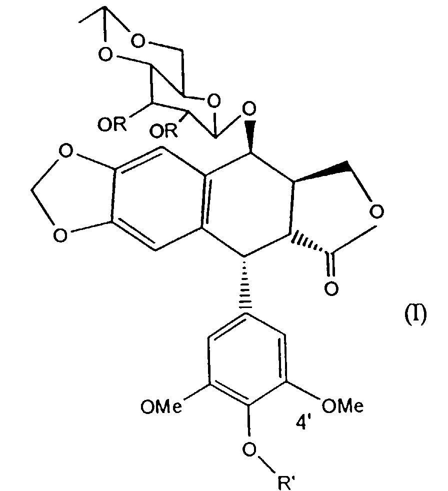 in which R' represents a phosphate monoester group; a carbamate group of -CO-N-(R₁R₂) type where N(R₁R₂) represents an aminodiacetic group or a polycyclic amine such as 3-aminoquinuclidine; a phosphonoacetic group CO-CH₂-PO₃H₂; CO-CH₂-SO₂CH₂COOH; CO-CH₂-OCH₂COOH; or a radical R of formula A-Z-CH₂-CO in which
• Z represents an oxygen or sulphur atom, an SO₂ group or a linear or branched C₁₋₄ alkylene, and
• A represents a substituted or unsubstituted phenyl ring, on condition that:
- in the case where R'=R, that is to say triacyl derivatives, A represents an aromatic ring possessing a salifiable function, except 4-hydroxyphenyl,
- in the case where R'≠R, A represents a benzyl, naphthyl, heteroaryl or phenyl residue which is substituted or unsubstituted, it being possible in this case for the phenyl to be substituted one, two, three, four or five times, irrespective of its position on the aromatic ring, with identical or different groups chosen from halogen, F, Cl, Br, linear or cyclic C1-C6 alkoxy, C₁-C₆ alkyl, methylenedioxy, OCF₃, CF₃, NO₂, CN, OCH₂ Aryl, OH, OPO₃H₂, CH₂-PO₃H₂, PO₃H₂, OCH₂CO₂H, COOH, CH₂COOH, COCH₃ and CHO groups,
as well as its salts with therapeutically acceptable and water-soluble, inorganic or organic acids or bases.

2. Compound according to Claim 1, characterized in that R' represents a phosphate monoester group (P03H2) or a carbamate group CONR₁R₂ in which NR₁R₂ represents an aminodiacetic group or a 3-aminoquinuclidine group, or CO-CH₂-PO₃H₂.

3. Compound according to Claims 1 and 2, characterized in that R is chosen from the radicals:
phenoxyacetyl, 3,4-methylenedioxyphenoxyacetyl, 4-methoxyphenoxyacetyl, 4-hydroxyphenoxyacetyl, 4-phosphonooxyphenoxyacetyl, 4-carboxymethylphenoxyacetyl, 4-carboxymethoxyphenoxyacetyl, 4-carboxyphenoxyacetyl, 4-trifluoromethylphenoxyacetyl, 4-trifluoromethoxyphenoxyacetyl, 4-chlorophenoxyacetyl, 4-nitrophenoxy-acetyl, 4-fluorophenoxyacetyl, cyclohexyloxyacetyl, phenylsulphonylacetyl, pentafluorophenoxyacetyl, 2 and 4 formylphenoxyacetyl, 4-cyanophenoxyacetyl.

4. Compound according to one of Claims 1 to 3, characterized in that it is chosen from organic or inorganic salts of:
• 4'-demethyl-4'-deoxy-4'-phosphate-4-O-(2,3-bis-phenoxyacetyl-4,6-ethylidene-β-D-glucosyl)epipodophyllotoxin,
• 4'-demethyl-4'-di(carboxymethyl)aminocarbonyl-4-O-(2,3-bisphenoxyacetyl-4,6-ethylidene-β-D-glucosyl)-epipodophyllotoxin,
• 4'-demethyl-4' -phosphonoacetyl-4-O-(2,3-bis-phenoxyacetyl-4,6-ethylidene-β-D-glucosyl)epipodophyllotoxin,
• 4'-demethyl-4'-deoxy-4'-phosphate-4-O-(2,3-bis(4-trifluoromethylphenoxyacetyl)-4,6-ethylidene-β-D-glucosyl)epipodophyllotoxin,
• 4'-demethyl-4'-di(carboxymethyl)aminocarbonyl-4-O-(2,3-bis(4-trifluoromethoxyphenoxyacetyl)-4,6-ethylidene-β-D-glucosyl)epipodophyllotoxin,
• 4'-demethyl-4'-deoxy-4'-phosphate-4-O-(2,3-bis(4-trifluoromethoxyphenoxyacetyl)-4,6-ethylidene-β-D-glucosyl)epipodophyllotoxin,
• 4'-demethyl-4'-phosphonoacetyl-4-O-(2,3-bis(4-trifluoromethoxyphenoxyacetyl)-4,6-ethylidene-β-D-glucosyl)epipodophyllotoxin,
• 4'-demethyl-4'-(4-phosphonooxyphenoxyacetyl)-4-O-(2,3-bis(4-phosphonooxyphenoxyacetyl)-4,6-ethylidene-β-D-glucosyl)epipodophyllotoxin,
• 4'-demethyl-4'-deoxy-4'-phosphate-4-O-(2,3-bis(4-phosphonooxyphenoxyacetyl)-4,6-ethylidene-β-D-glucosyl)epipodophyllotoxin,
• 4'-demethyl-4'-di(carboxymethyl)aminocarbonyl-4-O-(2,3-biscyclohexyloxyacetyl-4,6-ethylidene-β-D-glucosyl)epipodophyllotoxin,
• 4'-demethyl-4'-deoxy-4'-phosphate-4-O-(2,3-bis-cyclohexyloxyacetyl)-4,6-ethylidene-β-D-glucosyl)epipodophyllotoxin,
• 4'-demethyl-4'-(3-quinuclidinylaminocarbonyl)-4-O-(2,3-bis-(3,4-methylenedioxyphenoxyacetyl)-4,6-ethylidene-β-D-glucosyl)epipodophyllotoxin,
• 4'-demethyl-4'-deoxy-4'-phosphate-4-O-(2,3-bis-(3,4-methylenedioxyphenoxyacetyl)-4,6-ethylidene-β-D-glucosyl)epipodophyllotoxin,
• 4'-demethyl-4'-deoxy-4'-phosphate-4-O-(2,3-bis-(2,3,4,5,6-pentafluorophenoxyacetyl)-4,6-ethylidene,β-D-glucosyl)epipodophyllotoxin,
• 4'-demethyl-4'-deoxy-4'-phosphate-4-O-(2,3-bis(4-fluorophenoxyacetyl)-4,6-ethylidene-β-D-glucosyl)epipodophyllotoxin,
• 4'-demethyl-4'- (3-quniuclidinylaminocarbonyl)-4-O-(2,3-bis(4-chlorophenoxyacetyl)-4,6-ethylidene-β-D-glucosyl)epipodophyllotoxin,
• 4'-demethyl-4'-deoxy-4'-phosphate-4-O-(2,3-bis(4-chlorophenoxyacetyl)-4,6-ethylidene-β-D-glucosyl)epipodophyllotoxin,
• 4'-demethyl-4'-deoxy-4'-phosphate-4-O-(2,3-bis-phenylsulphonylacetyl)-4,6-ethylidene-β-D-glucosyl)epipodophyllotoxin,
• 4'-demethyl-4'-(4-carboxyphenoxyacetyl)-4-O-(2,3-bis(4-carboxyphenoxyacetyl)-4,6-ethylidene-β-D-glucosyl)epipodophyllotoxin,
• 4'-demethyl-4'-(4-carboxymethylphenoxyacetyl)-4-O-(2,3-bis(4-carboxymethylphenoxyacetyl)-4,6-ethylidene-β-D-glucosyl) epipodophyllotoxin,
• 4'-demethyl-4'-(4-carboxymethoxyphenoxyacetyl)-4-O-(2,3-bis(4-carboxymethoxyphenoxyacetyl)-4,6-ethylidene-β-D-glucosyl)epipodophyllotoxin,
• 4'-demethyl-4'-(3-quinuclidinylaminocarbonyl)-4-O-(2,3-bis(4-nitrophenoxyacetyl)-4,6-ethylidene-β-D-glucosyl) epipodophyllotoxin,
• 4'-demethyl-4'-deoxy-4'-phosphate-4-O-(2,3-bis(4-methoxyphenoxyacetyl)-4,6-ethylidene-β-D-glucosyl)epipodophyllotoxin,
• 4'-demethyl-4'-deoxy-4'-phosphate-4-O-(2,3-bis(4-cyanophenoxyacetyl)-4,6-ethylidene-β-D-glucosyl)epipodophyllotoxin,
• 4'-demethyl-4'-deoxy-4'-phosphate-4-O-(2,3-bis(4-nitrophenoxyacetl)-4,6-ethylidene-β-D-glucosyl)epipodophyllotoxin,
• 4'-demethyl-4'-deoxy-4'-phosphate-4-O-(2,3-bis(4-methylphenoxyacetyl)-4,6-ethylidene-β-D-glucosyl)epipodophyllotoxin,
• 4'-demethyl-4'-deoxy-4'-phosphate-4-O-(2,3-bis(4-hydroxyphenoxyacetyl)-4,6-ethylidene-β-D-glucosyl)epipodophyllotoxin.

5. Compounds according to Claim 4, characterized in that the agents salifying the acidic functions are more particularly chosen from amines such as N-methylglucamine, lysine and triethanolamine, or cations such as sodium or potassium, and in that the agents salifying the basic functions are more particularly chosen from inorganic or organic acids such as hydrochloric acid, sulphuric acid, methanesulphonic acid, ethanolsulphonic acid, maleic acid and tartaric acid.

6. Process for the preparation of a compound according to one of Claims 1 to 4, characterized in that a glycosylated intermediate of general formula II 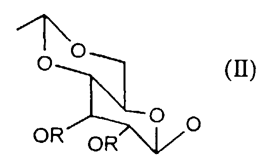 is reacted with an intermediate III 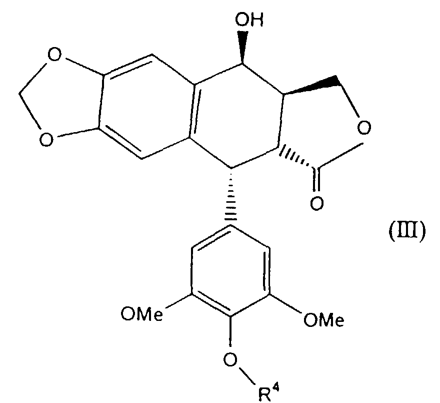 for which R is defined as in Claim 1 and R⁴ is a protecting group, for example benzyloxycarbonyl or a carbamate radical, which product is deprotected in order to obtain the compounds of formula I, R'=H, and which is reacted with suitable reagents in order to obtain the compounds of formula I, R'≠H.

7. Process for the preparation of a compound according to one of Claims 1 to 4, characterized in that etoposide protected in position 4' with a benzyloxycarbonyl or quinuclidene carbamate group of formula V, in which R⁴ is defined as in Claim 6, is reacted with an acylating reagent of A-Z-CH₂CO type to give, after hydrogenolysis or hydrolysis, the compound of formula I, such that R'=H.

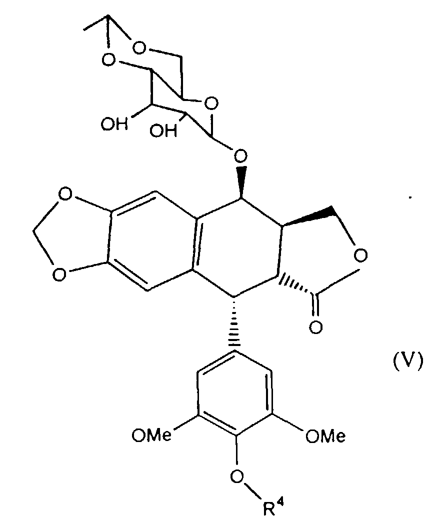

8. Process for the preparation of a compound according to one of Claims 1 to 4, characterized in that etoposide is reacted by triacylation in order to obtain the compounds of formula I where R'=R=A-Z-CH₂CO, A possessing a salifiable function.

9. Process for the preparation of the salt of a compound according to Claims 1 to 5, characterized in that the acidic compound is treated with a stoichiometric amount of base, or of ion exchange resin, and in that lyophilization or crystallization is carried out.

10. Process for the preparation of the salt of a compound according to Claims 1 to 5, characterized in that the basic compound is treated with a stoichiometric amount of the acidic agent and lyophilization or crystallization is carried out.

11. The compound according to one of Claims 1 to 5, as medicinal product.

12. Pharmaceutical composition, characterized in that it comprises at least one compound of formula I according to one of Claims 1 to 5, and a suitable excipient.

13. Use of a compound of formula I according to one of Claims 1 to 5, for the preparation of a medicinal product intended for anticancer treatment, in particular alveolar lung cancer, embryonic tumours, neuroblastomas, cancer of the kidney, paediatric tumours, hodgkimian and non-hodgkimian lymphomas, acute leukaemias, placental choriocarcinomas and mammary adenocarcinomas, as well as to increase the therapeutic efficacy of topoisomerase II-inhibitor compounds for the treatment of tumours which do not respond to the usual therapies: colorectal cancers and melanomas.

14. Use of a compound of formula I according to one of Claims 1 to 5, for the preparation of a medicinal product intended for the treatment of rheumatoid arthritis, and complaints caused by human papilloma virus.
